# EUROPEAN PATENT APPLICATION

(11) **EP 4 458 848 A1**
(43) Date of publication of application: **06.11.2024**
(21) Application number: 22916843.0
(22) Date of filing: 30.12.2022
(51) Int. Cl.: C07K 16/00, C07K 14/705, A61P 25/28, A61K 38/00, A61K 39/00

(54) **BLOOD-BRAIN BARRIER PERMEABLE FUSION PROTEIN AND USES THEREOF**

(30) Priority: 31.12.2021 KR 20210194319; 29.12.2022 KR 20220189752
(71) Applicant: ImNewRun, Inc., Suwon-si, Gyeonggi-do 16419 (KR)
(72) Inventor: KIM, Han Joo, Suwon-si, Gyeonggi-do 16419 (KR); LEE, Eun A, Suwon-si, Gyeonggi-do 16419 (KR); AN, Yong Il, Suwon-si, Gyeonggi-do 16419 (KR)
(74) Representative: Weickmann & Weickmann PartmbB
(86) International application number: PCT/KR2022/021730
(87) International publication number: WO 2023/128702

(57) **Abstract**

The present disclosure relates to a blood-brain barrier permeable fusion protein and uses thereof, and provides a blood-brain barrier permeable fusion protein, a polynucleotide encoding the fusion protein, a vector including the polynucleotide, a transfection cell line transfected with the vector, and a pharmaceutical composition for preventing or treating diseases associated with brain dysfunction, including the fusion protein as an active ingredient.

## Description

### Technical Field

The present disclosure relates to a blood-brain barrier permeable fusion protein and uses thereof, and also relates to a fusion protein including an IgG antibody and a binding moiety to a helical region of a transferrin receptor linked to termini of the IgG antibody, and medical use of the fusion protein. The present application is based on and claims priority to Korean Patent Application Nos. 10-2021-0194319, filed on December 31, 2021 and 10-2022-0189752, filed on December 29, 2022, in the Korean Intellectual Property Office, the disclosure of which is incorporated by reference herein in its entirety.

### Background Art

The blood-brain barrier (BBB) is a blood vessel barrier that separates the brain and the blood, and serves to isolate the central nervous system, including the brain, from potentially dangerous substances in the blood. The BBB is a cerebrovascular-centered structure composed of brain endothelial cells), astrocytes, pericytes, etc., and is distributed throughout the brain blood vessels within the brain tissue. The brain endothelial cells of the BBB are firmly bound together by tight junctions, and the astrocytes and astrocyte endfeet surround the periphery of the blood vessels, forming a barrier that selectively inhibits substances flowing along the blood inside the brain blood vessels from permeating the brain barrier and being absorbed/delivered to brain tissue. Such a barrier-like structure allows permeation of substances selectively, or inhibits the permeation, depending on the type and size of substances. In the case of water and oxygen that are essential for sustaining life, these substances can cross the BBB by diffusion, and in the case of amino acids or glucose used as energy sources, these substances can be delivered from the blood to the brain tissue by active transport. However, in the case of toxic substances, which can potentially affect the brain, and pathogens, etc., the transport of these substances is inhibited by the BBB, and even if they do penetrate the BBB, they are returned to the blood by the pumping action of cells, thereby preventing their absorption into the brain tissue. In this regard, the BBB serves to protect the brain tissue.

However, the BBB with this structure acts as a major obstacle in the pharmacological treatment of diseases associated with brain dysfunction, such as Alzheimer's disease, Parkinson's disease, brain cancer, etc. In fact, it has been reported that only a few therapeutic drugs with molecular weights of 400 Dalton to 500 Dalton are able to penetrate the BBB, and most therapeutic drugs are either unable to be delivered to the brain tissue by the BBB or are delivered in very small amounts. Either way, therapeutic drugs fail to exhibit their efficacy in the target organ, the brain tissue. To address these issues, several delivery technologies have been proposed, including lipid solubilization of drugs to directly cross the cell membrane of endothelial cells, delivery through the innate nutrient delivery system, and antibody-based delivery using receptor-mediated transcytosis. However, since these technologies act not only on the brain but also on other organs, there are concerns about side effects in the body, and there are limits to clinical use due to the difficulty in determining the appropriate concentration or content due to differences in delivery efficacy for each patient.

Against this technical background, various studies are underway to improve permeability to the BBB and selective delivery to the brain tissue (KR 10-2021-0005647), but these are still incomplete.

### Disclosure

### Technical Problem

To overcome these limitations of the prior art, the prevent inventors designed a fusion protein comprising: an IgG antibody; and a tetravalent binding moiety to a helical region of a transferrin receptor at the termini of the IgG antibody. It is found that, due to a specific expression pattern of transferrin receptor clusters in the brain vascular region and a resulting interaction specific with the clusters within the brain vascular region, not only the fusion protein improved the permeability to the blood-brain barrier and improved delivery efficiency to the brain tissue, but also the IgG antibodies were distributed at a high level selectively in the brain tissue compared to other tissues, thereby completing the present disclosure.

Accordingly, one aspect provides a blood-brain barrier permeable fusion protein, the fusion protein including: an IgG antibody; and a tetravalent binding moiety to a helical region of a transferrin receptor (TfR) linked to a C-terminus region of a light chain and a C-terminus region of a heavy chain of the IgG antibody.

Another aspect provides a polynucleotide encoding the fusion protein, a vector including the polynucleotide, and a transfection cell line transfected with the vector.

Another aspect provides a pharmaceutical composition for preventing or treating diseases associated with brain dysfunction, the pharmaceutical composition including the fusion protein as an active ingredient.

Other purposes and advantages of the present application will become more obvious with the following detailed description, claims, and drawing. Contents not described in the present specification will be sufficiently recognized and inferred by those skilled in the technical field of the present application or in a similar technical field therewith, and thus descriptions of such contents will be omitted.

### Technical Solution

One aspect provides a blood-brain barrier permeable fusion protein, the fusion protein including: an IgG antibody; and a tetravalent binding moiety to a helical region of a transferrin receptor (TfR) linked to a C-terminus region of a light chain and a C-terminus region of a heavy chain of the IgG antibody.

### Blood-brain barrier (BBB)

The term "blood-brain barrier (BBB)" as used in the present specification refers to a structure centered on the brain vessels, the structure enabling selective uptake of substances, such as amino acids, glucose, etc., essential to life support while strictly controlling the movement of substances, such as ions, molecules, and pathogens, present in the blood into brain tissue. The BBB consists of cells such as BBB endothelial cells, astrocytes, pericytes, etc. These cells are arranged in such a way that they share a common baseline membrane, and tightly connected endothelial cells are distributed on one side of the BBB, and astrocytes surrounding the perivascular areas are distributed on the other side of the BBB. The BBB endothelial cells constitute the walls of capillaries, and are connected by very strong and complex tight junctions. Such a structure forms a physical barrier and serves to inhibit the simple diffusion of most substances, wherein the most substances include average-sized molecules, such as insulin, to large-sized molecules. Also, astrocytes are a type of glial cells of the central nervous system that interacts with brain vessels to affect endothelial cell function, blood flow, and ion balance in the brain. Astrocytes use processes called end feet to surround blood vessels at one end and come into close contact with neurons at the synapse at the other end. The BBB with this structure is a major obstacle in pharmacological treatment since it not only protects brain tissue from potentially dangerous substances in the blood, but also disturbs the delivery of substances effective in treating diseases to brain tissue. Therefore, technologies that improve permeability to the BBB may improve the applicability or efficacy of therapeutic agents in the field of medicine targeting brain tissue.

### IgG antibody

The term "IgG antibody" as used in the present specification is an immunoglobulin molecule that is immunologically reactive with a specific antigen, and refers to a protein molecule that specifically recognizes an antigen and serves as a receptor for the antigen. The antibody has a heavy chain and a light chain, wherein each of the heavy chain and the light chain includes a constant region and a variable domain. The variable domain of each of the light chain and the heavy chain includes three variable domains called complementarity-determining regions (CDRs) and four framework regions. The CDR mainly functions to bind to an epitope of the antigen. The CDRs of each chain are typically called CDR1, CDR2, and CDR3 sequentially starting from the N-terminus, and are also identified by the chain on which a particular CDR is located. The antibody may include all of a polyclonal antibody, a monoclonal antibody, a full-length antibody, and an antibody fragment or antigen-binding fragment including an antigen-binding domain. A full-length antibody has a structure with two full-length light chains and two full-length heavy chains, wherein each light chain is connected to the heavy chain via a disulfide bond. The full-length antibody may preferably be IgG, and its subtypes may include IgG1, IgG2, IgG3, and IgG4.

Also, the antibody may be a natural antibody or a recombinant antibody. A natural antibody refers to an antibody that has not been genetically modified, and in this regard, the risk of immunogenicity that genetically modified antibody may have *in vivo* may be significantly lower. A recombinant antibody refers to an antibody that has been genetically modified, and in this regard, the recombinant antibody may have the ability to add antigen-binding force or desired characteristics through genetic modification.

The term "antigen-binding fragment" or "antibody fragment" as used in the present specification refers to a fragment that retains a function to bind to an antigen, and may include functional antibody fragments, such as Fab, F(ab'), F(ab')₂, Fv, single chain Fv (scFv), and scFv-Fc bivalent molecule, or a combination thereof. Also, for example, the antigen-binding fragment may include the following, but is not limited thereto, and structures of IgG-like bispecific antibodies known in the art may be employed without limitation: (1) Fab, which is a fragment that includes a monovalent antigen-binding fragment of an antibody molecule and can be produced by digesting the whole antibody with an enzyme, papain, to yield portions of an unprocessed light chain and one heavy chain; (2) Fab'; which is a fragment of an antibody molecule that can be obtained by treating the whole antibody with pepsin followed by reduction to yield portions of unprocessed light chains and heavy chains; two Fab' fragments obtained per antibody molecule; (3) (Fab')₂, which is a fragment of an antibody that can be obtained by treating the whole antibody with an enzyme, pepsin, without subsequent reduction, and is a dimer of two Fab' fragments bonded together by two disulfide bonds; (4) Fv, which is a genetically modified fragment including a variable domain of a light chain and a variable domain of a heavy chain that are expressed as two chains; (5) single chain antibody (SCA or scFv), which is a genetically fused single-chain molecule including a variable domain of a light chain and a variable domain of a heavy chain that are linked by a suitable polypeptide linker; (6) scFv-Fc, which is a molecule produced by fusing a single chain Fv (scFv) with a hinge region from immunoglobulins (Ig) such as IgG and Fc regions.

Also, the antibody or antibody fragment is an immunoglobulin molecule that retains the function to bind to an antigen, and may include a monoclonal antibody, a multi-specific antibody, a human antibody, a humanized antibody, a mouse antibody, a chimeric antibody, scFV, a single chain antibody, a Fab fragment, a F(ab') fragment, a F(ab')₂, disulfide-linked Fvs (sdFV), a scFv fragment, a scFv-Fc fragment, a Fv fragment, a diabody, triabody, a tetrabody, etc. Fab among the antibody fragments has a structure with variable domains of the light and heavy chains, a constant region of the light chain, and a first constant region (CH1) of the heavy chain, and has one antigen-binding site. Fab' differs from Fab in that it has a hinge region including one or more cysteine residues at the C-terminus of a CH1 domain of the heavy chain. A F(ab')₂ antibody is produced by forming disulfide bonds between cysteine residues at the hinge region of Fab'. Fv is the smallest antibody fragment including only a heavy chain variable domain and a light chain variable domain. Two-chain Fv (two-chain Fv) consists of a heavy chain variable domain and a light chain variable domain connected by a non-covalent bond. These antibody fragments may be obtained by using proteolytic enzymes (for example, Fab may be obtained by papain digestion of whole antibody, and F(ab')₂ may be obtained by pepsin cleavage), or may be also prepared through genetic recombination technologies.

In the present specification, the IgG antibody is a subject that forms a fusion with a binding moiety to a helical region of a transferrin receptor, and in this regard, each of the light chain C-terminus and the heavy chain C-terminus constituting the IgG antibody may be linked to or conjugated with a binding moiety to a helical region of a transferrin receptor. The IgG antibody may have enhanced selective permeability of the BBB, and may be distributed at a relatively high level in brain tissue compared to other organs and cells.

In an embodiment, the IgG antibody may be an antibody or antigen-binding fragment capable of binding to a target antigen known in the art, applicable to treating, alleviating, or detecting diseases associated with brain dysfunction. For example, the IgG antibody may be a pharmaceutically active ingredient for treating or alleviating diseases associated with brain dysfunction. The IgG antibody may be an antibody to treat or alleviate diseases associated with brain dysfunction, such as Alzheimer's disease; dementia with Lewy bodies; frontotemporal dementia; tangle only dementia; Parkinson's disease; multiple sclerosis; amyotrophic lateral sclerosis (ALS); traumatic brain injury; progressive supranuclear palsy; corticobasal degeneration; globular glial tauopathy; aging-related tau astrogliopathy; chronic traumatic encephalopathy (CTE); brain cancer such as primary CNS lymphoma (PCNSL), glioma, neuroblastoma, metastatic brain tumor, meningioma, etc.; Pick's disease; anti-IgLON5-related taupathy; Guadeloupean parkinsonism; nodding syndrome; pain; epilepsy; autism; stroke; Guillain-Barre syndrome (GBS); Creutzfeldt-Jakob disease (CJD); Huntington's disease; progressive multifocal leukoencephalopathy (PML); depression; post-traumatic stress disorder (PTSD); and lysosomal storage disease (LSD). For example, the IgG antibody may be αPD-L1 IgG antibody, anti-PD1 IgG antibody, anti-Tau IgG antibody, anti-HER2 IgG antibody, anti-Aβ IgG antibody, anti-TDP-43 IgG antibody, anti-alpha-synuclein IgG antibody, anti-SIGLEC3 IgG antibody, or anti-TREM2 IgG antibody, but is not limited thereto.

### Transferrin receptor (TfR)

The term "transferrin receptor (TfR)" as used in the present specification refers to a membrane glycoprotein expressed on the surface of cells that mediate intracellular uptake of iron from transferrin which is a plasma glycoprotein. TfRs are widely distributed on normal cells in a variety of tissues and have been reported to be expressed in large amounts on activated immune cells and tumor cells. Therefore, TfR-mediated delivery technologies require not only effective delivery to brain tissue, but also low delivery to organs other than brain tissue and to normal cells. In fact, TfR-mediated carriers or therapeutic agents have been reported to cause red blood cell-related toxicities, including decreased number of reticulocytes, severe coma, intermittent limb stiffness, general stiffness, hemolysis, and hemoglobinuria. Also, the extracellular domain (ectodomain) of the TfR is divided into an apical domain, a helical domain, and a protease-like domain, and these are known to have different binding affinities to target substances during receptor-mediated transcytosis.

The genetic information of the TfR can be obtained from the known databases such as GenBank of the National Center for Biotechnology Information (NCBI). For example, the TfR may consist of an amino acid sequence of SEQ ID NO: 1, but is not limited.

### Transferrin receptor (TfR) cluster

The term "transferrin receptor (TfR) cluster" as used in the present specification refers to a cluster in which a plurality of TfRs densely present in a localized area, wherein the transferrin receptor cluster may be distributed in cells or tissues such as blood vessels, in the form of an aggregation of a plurality of transferrin receptors. In particular, unlike TfR clusters distributed in other organ tissues or cells, the TfR clusters present in the brain vascular region may have a specific expression pattern. Specifically, the specific expression pattern refers to a significantly dense distribution of TfRs within a localized region where clusters are formed, compared to patterns in other organs or cells. These brain tissue-specific cluster and expression pattern of the TfR within the cluster may be closely correlated with high permeability to the BBB and selective delivery to brain tissue.

### Binding moiety to helical region of TfR

The term "binding moiety to the helical region of the TfR" as used in the present specification refers to a functional unit that interacts with a receptor present on the surface of cells constituting the BBB, and specifically, may refer a moiety that has effective binding affinity for a helical region among regions constituting the TfR. The binding moiety to the helical region of the TfR may be in a tetravalent form, reflecting the expression pattern of the TfR clusters specific in brain tissue blood vessels. Here, the helical region of the TfR is a functional/structural region exposed to the outside such that valid tetravalent bonds are formed between fusion proteins and a plurality of TfRs densely distributed in a cluster of blood vessels in brain tissue, and It may contribute to high permeability to the BBB and selective delivery to brain tissue. Therefore, the binding moiety capable of binding to the helical region of the TfR is linked to the IgG antibody, thereby not only significantly improving permeability of the IgG antibody to the BBB, but also conferring functionality to enable selective delivery/uptake of the IgG antibody into brain tissue.

The binding moiety is intended to interact with the TfR clusters distributed in vascular regions of brain tissue to form a complex/fusion, and may have binding affinity with a plurality of TfRs within the localized region where the TfR clusters are formed. In detail, the binding moiety may have binding affinity with the helical region of the TfR, specifically, with at least one amino acid selected regions from the 606^{th} amino acid to the 665^{th} amino acid based on the TfR of SEQ ID NO: 1.

In an embodiment, the binding moiety to the helical region of the TfR may be linked to the C-terminus of the light chain and the C-terminus of the heavy chain of the IgG antibody, and in this regard, the binding moiety to the helical region of the TfR may be linked to the IgG antibody in a tetravalent form, thereby exhibiting the intended functions.

In an embodiment, the binding moiety to the helical region of the TfR may have 6 amino acids to 250 amino acids in length. For example, the binding moiety to the helical region of the TfR may have, in length, 6 amino acids to 240 amino acids, 6 amino acids to 220 amino acids, 6 amino acids to 200 amino acids, 6 amino acids to 180 amino acids, 6 amino acids to 160 amino acids, 6 amino acids to 140 amino acids, 6 amino acids to 120 amino acids, 6 amino acids to 100 amino acids, 6 amino acids to 80 amino acids, 6 amino acids to 60 amino acids, 6 amino acids to 40 amino acids, 6 amino acids to 20 amino acids, 6 amino acids to 10 amino acids, 10 amino acids to 250 amino acids, 10 amino acids to 240 amino acids, 10 amino acids to 220 amino acids, 10 amino acids to 200 amino acids, 10 amino acids to 180 amino acids, 10 amino acids to 160 amino acids, 10 amino acids to 140 amino acids, 10 amino acids to 120 amino acids, 10 amino acids to 100 amino acids, 10 amino acids to 80 amino acids, 10 amino acids to 60 amino acids, 10 amino acids to 40 amino acids, 10 amino acids to 20 amino acids, 20 amino acids to 250 amino acids, 20 amino acids to 240 amino acids, 20 amino acids to 220 amino acids, 20 amino acids to 200 amino acids, 20 amino acids to 180 amino acids, 20 amino acids to 160 amino acids, 20 amino acids to 140 amino acids, 20 amino acids to 120 amino acids, 20 amino acids to 100 amino acids, 20 amino acids to 80 amino acids, 20 amino acids to 60 amino acids, 20 amino acids to 40 amino acids, 40 amino acids to 250 amino acids, 40 amino acids to 240 amino acids, 40 amino acids to 220 amino acids, 40 amino acids to 200 amino acids, 40 amino acids to 180 amino acids, 40 amino acids to 160 amino acids, 40 amino acids to 140 amino acids, 40 amino acids to 120 amino acids, 40 amino acids to 100 amino acids, 40 amino acids to 80 amino acids, 40 amino acids to 60 amino acids, 60 amino acids to 250 amino acids, 60 amino acids to 240 amino acids, 60 amino acids to 220 amino acids, 60 amino acids to 200 amino acids, 60 amino acids to 180 amino acids, 60 amino acids to 160 amino acids, 60 amino acids to 140 amino acids, 60 amino acids to 120 amino acids, 60 amino acids to 100 amino acids, or 60 amino acids to 80 in length, but the length is not limited thereto.

Also, the binding moiety to the helical region of the TfR may have, in length, for example, 10 amino acids to 48 amino acids, 10 amino acids to 44 amino acids, 10 amino acids to 40 amino acids, 10 amino acids to 36 amino acids, 10 amino acids to 32 amino acids, 10 amino acids to 28 amino acids, 10 amino acids to 24 amino acids, 10 amino acids to 20 amino acids , 10 amino acids to 16 amino acids, 10 amino acids to 12 amino acids, 12 amino acids to 48 amino acids, 12 amino acids to 44 amino acids, 12 amino acids to 40 amino acids, 12 amino acids to 36 amino acids, 12 amino acids to 32 amino acids, 12 amino acids to 28 amino acids, 12 amino acids to 24 amino acids, 12 amino acids to 20 amino acids, 12 amino acids to 16 amino acids, 14 amino acids to 48 amino acids, 14 amino acids to 44 amino acids, 14 amino acids to 40 amino acids, 14 amino acids to 36 amino acids, 14 amino acids to 32 amino acids, 14 amino acids to 28 amino acids, 14 amino acids to 24 amino acids, 14 amino acids to 20 amino acids, or 14 amino acids to 16 amino acids, but the length is not limited thereto.

In an embodiment, the binding moiety to the helical region of the TfR may include an amino acid sequence in which a monomeric unit sequence is repeated 2 times to 5 times, 2 times to 4 times, or 2 times to 3 times. The monomeric unit sequences may be connected together by fusion or via linker peptides. The linker peptide may have 2 amino acids to 50 amino acids in length. For example, the linker peptide may have, in length, 2 amino acids to 40 amino acids, 2 amino acids to 30 amino acids, 2 amino acids to 20 amino acids, 2 amino acids to 15 amino acids, 2 amino acids to 10 amino acids, 2 amino acids to 5 amino acids, 5 amino acids to 50 amino acids, 5 amino acids to 40 amino acids, 5 amino acids to 30 amino acids, 5 amino acids to 20 amino acids, 5 amino acids to 15 amino acids, or 5 amino acids to 10 amino acids, but the length is not limited thereto. Also, the linker peptide may be, for example, (GₗSₘ)ₙ (wherein I may be 2 to 8, m may be 1 to 5, and n may be 1 to 5), (G_{d}SₑAS)_{f} (wherein d may be 2 to 8, e may be 1 to 5, and f may be 1 to 5), (G₄S)ₐ(EAAAK)_{b}(G₄S)ₐ (wherein a and b may each be an integer from 1 to 4), or [(G₄S)ₚ(EAAAK)_{q}]ᵣ (wherein p and q may each be an integer from 1 to 4r), but is not limited thereto.

In an embodiment, the monomeric unit sequence constituting the binding moiety to the helical region of the TfR may include a cell-permeable peptide. The monomeric unit sequence may be, for example, any one of SEQ ID NOs: 3 to 43, but may be extended in a non-limiting manner as long as it has a valid interaction (hydrogen bonding, electrostatic attraction, van der Waals force), i.e., valid binding affinity, with the helical region of the TfR.

In an embodiment, each of a plurality of binding moieties to the helical region of the TfR may be identical to or different from each other.

### Blood-brain barrier (BBB) permeable fusion protein

The term "fusion protein" as used in the present specification refers to a protein formed through combination of two or more originally distinct proteins or parts thereof, and may optionally include a linker or space located between the two or more proteins. The term "BBB permeable fusion protein" as used in the present specification is a protein formed through binding between the IgG antibody and the binding moiety to the helical region of the TfR, and may refer to a general term for proteins having functional structures that allow selective delivery or uptake of an effective amount of IgG antibodies by penetration into the BBB. The functional structure refers to a structure modified to have effective binding affinity by reflecting the structure and expression pattern of the TfR clusters of vascular endothelial cells that are specifically distributed in the BBB, and may include the binding moiety to the helical region of the TfR and a tetravalent bond (link) between the binding moiety and the C-terminus regions of the heavy and light chains constituting the IgG antibody.

In an embodiment, the BBB permeable fusion protein may have a functional structure in which a tetravalent binding moiety to the helical region is linked to the C-terminus regions of the light chain and the C-terminus regions of the heavy chain of the IgG antibody.

In an embodiment, in the IgG antibody, the linkage between the helical region binding moiety and the C-terminus regions of the light chain and the linkage between the helical region binding moiety and the C-terminus regions of the heavy chain may be achieved by fusion to the terminus regions of the IgG antibody, or may be achieved via linker peptides. Here, the linker peptides are the same as described above.

In an embodiment, the BBB permeable fusion protein may form a complex by binding to the TfR that forms the TfR clusters specifically distributed in blood vessels of the BBB. For example, the BBB permeable fusion protein may bind to a plurality of the TfRs that are densely distributed within the TfR clusters, rather than to other organ tissues or cells.

According to an embodiment, the functional structure is designed to reflect the effective binding affinity with the helical region of the TfR and the specific distribution/expression pattern of the TfR clusters distributed in the cerebrovascular domains, and in this regard, a fusion protein in which a plurality of the binding moieties to the helical region of the TfR are linked to the C-termini of the light and heavy chains, i.e., a total of four terminus regions, of the IgG antibody is prepared. Afterwards, when an agent including the fusion protein is administered intravenously, not only was the delivery of the fusion protein including the IgG antibody to brain tissue significantly enhanced, but also the IgG antibody was distributed at a higher level in brain tissue than in other organs. In particular, effects of the fusion protein on normal cells including reticulocytes and other organ tissues were confirmed to be minimal. In addition, regarding a function derived from the tetravalent moiety with valid binding affinity with the helical region of the TfR and the functional structure including the tetravalent moiety, it was confirmed that the fusion protein exhibited effects such as high permeability to the BBB, regardless of types of the IgG antibody and specific sequences of the binding moiety. Therefore, in an embodiment, a functional structure that is linked to the binding moiety, enhances permeability of the IgG antibody to the BBB and simultaneously induces selective delivery of the IgG antibody to brain tissue, and is capable of contributing to reducing side effects of antibody-based drugs is newly identified.

In an embodiment, the BBB permeable fusion protein may include: a first binding moiety linked to the C-terminus region of the light chain of the IgG antibody; and a second binding moiety linked to the C-terminus region of the heavy chain of the IgG antibody.

In an embodiment, the BBB permeable fusion protein may be formulated for administration, and the administration may be by any non-limiting method of administering a conventional fusion protein or an antibody-based agent. For example, the administration may be intravenous administration, intramuscular administration, subcutaneous administration, intraperitoneal administration, ocular administration, intrathecal administration, intracerebroventricular administration, intranasal administration, etc. Under such administration conditions, the fusion protein may allow the IgG antibody as a fusion partner to be delivered/distributed to high levels within brain tissue, through an appropriate level of interaction with some regions of receptors present on the surface of cells constituting the BBB, i.e., the helical region of the TfR, or via linkage with the binding moiety to the helical region of the TfR that can provide binding affinity.

Another aspect provides: a polynucleotide encoding the fusion protein; a vector including the polynucleotide; and a transfection cell line transfected with the vector.

Among the terms or elements mentioned in the following polynucleotide, vector, or transfection cell line, those that have been already mentioned in the description of the BBB permeable fusion protein are the same as described above.

### Polynucleotides encoding fusion protein

The polynucleotide may be in the form of RNA or DNA, and DNA may include cDNA and synthetic DNA. DNA may be single-stranded or double-stranded. In the case of a single strand, it may be a coding strand or a non-coding (antisense) strand, and the coding sequence may, as a result of degeneracy or redundancy of the genetic code, may encode the same polypeptide.

The polynucleotide may also include a variant of the polynucleotide described herein, wherein the variant of the polynucleotide may be a naturally occurring allelic variant of the polynucleotide or a non-naturally occurring variant of the polynucleotide. The allelic variant may be an alternate form of a polynucleotide sequence that may have substitution, deletion, or addition of one or more nucleotides, without substantially changing the function of a polynucleotide to be encoded. It is well known in the art that a single amino acid may be encoded by one or more nucleotide codons and that the polynucleotide may be easily modified to prepare an alternate polynucleotide encoding the same peptide.

### Vector including polynucleotide

The term "vector" as used in the present specification is a DNA vehicle that allows expression of a target protein in a suitable host cell, and also refers to a genetic construct including regulatory elements operably linked to express a gene insert. A vector according to an embodiment may include expression regulatory factors, such as a promoter, an operator, a start codon, a stop codon, a polyadenylation signal, and/or an enhancer, and the promoter of the vector may be constitutive or inducible. In addition, the vector may be an expression vector capable of stably expressing the fusion protein in a host cell. For the expression vector, a conventional vector in the art used to express a foreign protein in plants, animals, or microorganisms may be used. The recombinant vector may be constructed through various methods known in the art. For example, the vector may include a selectable marker for selecting a host cell including a vector, and in the case of a replicable vector, it may include an origin of replication. In addition, the vector may be self-replicated or introduced into host DNA, wherein the vector may be selected from the group consisting of a plasmid, a lentivirus, an adenovirus, an adeno-associated virus, a retrovirus, a herpes simplex virus, and a vaccinia virus.

In an embodiment, the vector may include a promoter operable in animal cells, preferably, mammalian cells. A suitable promoter according to an embodiment may include promoters derived from mammalian viruses and promoters derived from the genome of mammalian cells, and examples thereof may include a cytomegalovirus (CMV) promoter, a T7 promoter, a U6 promoter, an H1 promoter, a murine leukemia virus (MLV)-long terminal repeat (LTR) promoter, an adenovirus early promoter, an adenovirus late promoter, a vaccinia virus 7.5K promoter, an SV40 promoter, a tk promoter of HSV, an RSV promoter, an EF1 alpha promoter, a metallothionine promoter, a beta-actin promoter, a promoter of human IL-2 gene, a promoter of human IFN gene, a promoter of human IL-4 gene, a promoter of human lymphotoxin gene, a promoter of human GM-CSF gene, a human phosphoglycerate kinase (PGK) promoter, a mouse PGK promoter, a surviving promoter, and a Chinese hamster elongation factor-1α(CHEF1-α) promoter.

In addition, in the vector, a polynucleotide sequence encoding the BBB permeable fusion protein may be operably linked to the promoter. The term "operably linked" as used in the present specification refers to a functional linkage between an regulatory sequence for the nucleic acid expression control sequence (e.g., a promoter, a signal sequence, or an array of transcriptional regulator binding sites) and another nucleic acid sequence. In this regard, the regulatory sequence may regulate transcription and/or translation of the another nucleic acid sequence.

### Transfection cell line

The term "transfection" as used in the present specification refers to a molecular biology technology in which a DNA chain fragment or a plasmid having foreign genes of a different kind from those of the original cell penetrates between cells and binds to the DNA existing in the original cell, thereby transforming the genetic traits of the original cell. The transfection refers to production of the BBB permeable fusion protein by inserting a polynucleotide encoding the BBB permeable fusion protein, or a vector including the polynucleotide into a host cell.

The transfection cell line or host cell may be preferably any one selected from the group consisting of: microorganisms including prokaryotes such as bacteria and eukaryotes such as yeast; insect-derived cells such as Sf9 cells; mouse-derived cells such as CHO cells; human-derived cells such as HEK293; and antibody-producing hybridomas, but is not limited thereto.

Another aspect provides: a pharmaceutical composition for preventing or treating a disease associated with brain dysfunction, the pharmaceutical composition including the fusion protein as an active ingredient; medical use of the fusion protein for preventing or treating a disease associated with brain dysfunction; or a method of treating a disease associated with brain dysfunction, the method including administering the pharmaceutical composition to a subject.

Among the terms or elements mentioned in the following pharmaceutical composition for preventing or treating a disease associated with brain dysfunction, medical use thereof, or method of treating a disease associated with brain dysfunction, those that have been already mention in the description of the BBB permeable fusion protein are the same as described above.

### Pharmaceutical composition for preventing or treating disease associated with brain dysfunction

The term "prevention" as used in the present specification refers to all actions that suppress or delay the onset of a disease associated with brain dysfunction by administering the pharmaceutical composition.

The term "treatment" as used in the present specification refers to all actions by which symptoms of a disease associated with brain dysfunction are ameliorated or beneficially changed by administering the pharmaceutical composition.

The "disease associated with brain dysfunction," which is a disease to be prevented or treated by the pharmaceutical composition, may include: Alzheimer's disease; dementia with Lewy bodies; frontotemporal dementia; tangle only dementia; Parkinson's disease; multiple sclerosis; amyotrophic lateral sclerosis; traumatic brain injury; progressive supranuclear palsy; corticobasal degeneration; globular glial tauopathy; aging-related tau astrogliopathy; chronic traumatic encephalopathy; brain cancer such as primary CNS lymphoma, glioma, neuroblastoma, metastatic brain tumor, meningioma, etc.; Pick's disease; anti-IgLON5-related taupathy; Guadeloupean parkinsonism; nodding syndrome; pain; epilepsy; autism; stroke; Guillian-Barre syndrome; Creutzfeldt-Jakob disease; Huntington's disease; progressive multifocal leukoencephalopathy; depression; post-traumatic stress disorder; and lysosomal storage disease, but is not limited thereto.

The pharmaceutical composition, i.e., a pharmaceutical preparation including a fusion protein, may be prepared by mixing an antibody having the desired degree of purity with any pharmaceutically acceptable carrier, excipient, or stabilizer (Remington's Pharmaceutical Sciences 16th edition, Osol, A. Ed. (1980)) in the form of a lyophilized preparation or an aqueous solution. Acceptable carriers, excipients, or stabilizers are nontoxic to recipients at the dosages and concentrations used, and may include: buffers such as phosphate, citrate, and other organic acids; antioxidants such as ascorbic acid and methionine; preservatives (such as octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as serum albumin, gelatin, or immunoglobulin; hydrophilic polymers such as polyvinylpyrrolidone; monosaccharides, disaccharides, and other carbohydrates such as glucose, mannose, or dextrins; chelating agents such as EDTA; sugars such as sucrose, mannitol, trehalose or sorbitol; salt-forming counterions such as sodium; metal complexes (e.g., Zn-protein complexes); and/or non-ionic surfactants such as Tween^{™}, PLURONICS^{™}, or polyethylene glycol (PEG).

The pharmaceutical composition may further include an activity ingredient, i.e., an optional ingredient with complementary activities that do not adversely affect each other. The type and effective amount of the pharmaceutical composition may be for example, determined depending on the amount of antibodies present in the preparation and the clinical parameters of the subject. The active ingredient may be, for example, delivered in the form of microcapsules such as hydroxymethylcellulose or gelatin-microcapsules and poly-(methylmethacrylate) microcapsules. wherein the active ingredient may be delivered to a colloidal drug delivery system (e.g., liposomes, albumin microspheres, microemulsions, nanoparticles, and nanocapsules) or may be delivered in an encapsulated form by microemulsion.

The pharmaceutical composition may be formulated, dosed, and administered in a manner consistent with medical practice. Factors to consider in this regard may include a specific disorder being treated, a specific mammal being treated, a clinical condition of an individual patient, a cause of the disorder, a site of delivery of an agent, a method of administration, a schedule of administration, and other factors known to a physician. The antibody may, but need not, be formulated in combination with one or more agents currently used for the prevention or treatment of a disease. The effective amount of the other agents is determined by the amount of antibodies present in the formulation, the type of disorder or treatment, and other factors. The pharmaceutical composition may be generally administered at the same dosages and by routes of administration as described herein, at about 1% to 99% of the dosages described herein, or at any dosage and by any route empirically/clinically determined to be appropriate.

### Method of treating disease associated with brain dysfunction

The method of treating a disease associated with brain dysfunction may use the pharmaceutical composition alone or in combination with other agents. For example, the pharmaceutical composition may be co-administered with at least one additional therapeutic agent. The additional therapeutic agent is a therapeutic agent effective in the treatment of a disease associated with brain dysfunction, and may include the following, but is not limited to: a cholinesterase inhibitor (e.g. donepezil, galantamine, rivastigmine, and tacrine), an NMDA receptor antagonist (e.g. memantine), an amyloid beta peptide aggregation inhibitor, an antioxidant, a γ-secretase modulator, a nerve growth factor (NGF) mimic or an NGF gene therapy agent, a PPARγ agonist, a HMS-CoA reduction enzyme inhibitor (statin), ampakine, a calcium channel blocker, a GABA receptor antagonist, a glycogen synthase kinase inhibitor, an intravenous immunoglobulin, a muscarinic receptor agonist, a nicotinic receptor modulator, active or passive amyloid beta peptide immunization, a phosphodiesterase inhibitor, a serotonin receptor antagonist, and an anti-amyloid beta peptide antibody.

Also, for the prevention or treatment of a target disease, the appropriate dosage of the pharmaceutical composition (when used alone or in combination with one or more other additional therapeutic agents) may be determined depending on the type of a disease being treated, the severity and course of the disease, whether an antibody is administered for prophylactic or therapeutic purposes, depending on the previously administered therapy, the clinical history of a patient and response to antibodies, and the judgment of the attending physician. The antibody may be suitably administered to a patient at once or over a series of treatments, and for the purpose of the present disclosure, the antibody may be provided in the form of a fusion protein bound to the tetravalent binding moiety to the helical region of the TfR. Depending on the type and severity of the disease, approximately 1 µg/kg to 100 mg/kg (e.g., 0.1 mg/kg to 100 mg/kg) of the antibody or fusion protein may be, for example, an initial candidate dose for administration to the patient, regardless of whether the administration is one or more separate administrations or whether the administration is by continuous injection. A typical daily dosage may be in a range of about 1 µg/kg to about 100 mg/kg or more depending on the aforementioned factors. For repeated administration over a period of several days or longer, depending on the pathology, the treatment will generally continue until the required suppression of disease symptoms occurs. One exemplary dosage of the antibody or fusion protein may be in a range of about 0.05 mg/kg to about 100 mg/kg. Accordingly, one or more doses of about 0.5 mg/kg, 2.0 mg/kg, 4.0 mg/kg, 10 mg/kg, or 20 mg/kg (or any combination thereof) may be administered to the patient. The dosage may be administered intermittently, for example, every week or every three weeks (for example, such that the patient is administered with a dosage of the antibody or fusion protein of about 2 mg/kg to about 20 mg/kg, or for example, about 6 mg/kg). Following the high loading dose at the beginning, one or more lower doses may be administered.

The term "subject" as used in the present specification refers to a target in need of disease treatment, and more particularly, refers to a mammal including a human or a non-human primate, such as a mouse, a dog, a cat, a horse, and a cow.

### Advantageous Effects

Regarding the blood-brain barrier permeable fusion protein according to one aspect, it was confirmed that, when a formulation including the fusion protein is administered intravenously, the delivery of IgG antibodies in the brain tissue is significantly enhanced due to valid interactions between the binding moieties and the transferrin receptors.

Also, regarding the blood-brain barrier permeable fusion protein according to one aspect, it was confirmed that, when a formulation including the fusion protein is administered intravenously, the fusion protein exhibited good biosafety as it can selectively deliver the IgG antibody at a high level in the brain tissue compared to the delivery to other organs and cells due to distribution of TfR clusters specific in the vascular region of the brain tissue and valid interaction with the clusters.

Therefore, the fusion protein according to one aspect can be used as an active ingredient in the pharmaceutical composition for the medical field requiring selective drug delivery to the brain tissue, for example, for the prevention or treatment of diseases associated with brain dysfunction.

### Description of Drawings

FIG. 1 is an image of a transferrin receptor obtained by cryogenic electron microscopy, showing the results of identifying a helical region within the transferrin receptor.
FIG. 2 shows the results of structural identification of bonds between a helical region binding moiety and a helical region of the transferrin receptor according to cryogenic electron microscopy.
FIG. 3 shows the thermodynamic structural stability of binding between the transferrin receptor and helical region binding moiety #01 according to an embodiment.
FIG. 4 shows the results of confirming levels of intracellular delivery of a helical region binding moiety #01 according to an embodiment, by using a human brain endothelial cell line.
FIG. 5 shows the results of confirming levels of IgG1 antibodies in brain tissue after intravenous administration of a blood-brain barrier permeable fusion protein F3#01 according to an embodiment to an animal model, by using a human IgG1 ELISA kit.
FIG. 6 shows the results of confirming levels of IgG1 antibodies in ISF after intravenous administration of a blood-brain barrier permeable fusion protein F3#01 according to an embodiment to an animal model, by using a human IgG1 ELISA kit.
FIG. 7 shows the results of real-time imaging of transcytosis of a fusion protein in the blood-brain barrier after intravenous administration of a blood-brain barrier permeable fusion protein F3#01 according to an embodiment to an animal model with a cranial window.
FIG. 8 shows the results of real-time confirmation of levels of a fusion protein present in an intravascular region (ROI 1), a blood vessel wall (ROI 2), and an extravascular region (ROI 3) after intravenous administration of a blood-brain barrier permeable fusion protein F3#01 according to an embodiment to an animal model with a cranial window.
FIG. 9 shows comparison results of levels of IgG1 antibodies in brain tissue, quantified as relative values to the control group, after intravenous administration of blood-brain barrier permeable fusion proteins (F1#01, F3#01, and F5#01) according to an embodiment to an animal model.
FIG. 10 shows the binding of the fusion proteins to the transferrin receptor confirmed by immunostaining after intravenous administration of blood-brain barrier permeable fusion proteins (F1#01, F3#01, and F5#01) according to an embodiment to an animal model, wherein (a) of FIG. 10 shows the results of the fusion protein F5#01, (b) of FIG. 10 shows the results of the fusion protein F1#01, and (c) of FIG. 10 shows the results of the fusion protein F3#01.
FIG. 11 shows comparison results of quantified levels of binding between the fusion proteins and the transferrin receptor after intravenous administration of blood-brain barrier permeable fusion proteins (F1#01, F3#01, and F5#01) according to an embodiment to an animal model.
FIG. 12 shows the results of interaction between a blood-brain barrier permeable fusion protein F3#01 according to an embodiment and a transferrin receptor, as confirmed by negative staining TEM analysis.
FIG. 13 shows the results of imaging of the structure of a complex formed by bonds between a blood-brain barrier permeable fusion protein according to an embodiment and a transferrin receptor.
FIG. 14 shows the results of confirming distribution levels of IgG1 antibodies in each organ after intravenous administration of a blood-brain barrier permeable fusion protein F3#01 according to an embodiment to an animal model, by using a human IgG1 ELISA kit.
FIG. 15 shows the results of confirming distribution levels of IgG1 antibodies in each organ after intravenous administration of a blood-brain barrier permeable fusion protein F3#01 according to an embodiment to an animal model, by using a human IgG1 ELISA kit.
FIG. 16 shows the results of confirming percentage (%) of reticulocytes among total red blood cells after a blood-brain barrier permeable fusion protein F3#01 according to an embodiment was intravenously administered to an animal model and blood was obtained.
FIG. 17 shows the results of confirming the concentration of the fusion protein in plasma after intravenous administration of a blood-brain barrier permeable fusion protein F3#01 according to an embodiment to an animal model.
FIG. 18 shows the results of calculating the blood-to-plasma ratio after treating plasma and blood samples with a blood-brain barrier permeable fusion protein F3#01 according to an embodiment.
FIG. 19 shows the results of confirming levels of IgG1 antibodies in brain tissue after intravenous administration of a blood-brain barrier permeable fusion protein F3#01 according to an embodiment to an animal model, by using a human IgG1 ELISA kit.
FIG. 20 shows the results of confirming distribution levels of IgG1 antibodies in each organ after intravenous administration of a blood-brain barrier permeable fusion protein F3'#01 according to an embodiment to an animal model, by using a human IgG1 ELISA kit.
FIG. 21 shows the results of confirming distribution levels of IgG1 antibodies in each organ after intravenous administration of a blood-brain barrier permeable fusion protein F3'#01 according to an embodiment to an animal model, by using a human IgG1 ELISA kit.
FIG. 22 shows the results of comparing amounts of a transferrin receptor expressed in brain tissue, vascular cells, liver, lung, kidney, and spleen, through Western blotting.
FIG. 23 shows the results of confirming an expression pattern of a transferrin receptor in brain tissue, liver, spleen, lung, and reticulocytes, through confocal microscopy images and super-resolution STED confocal microscopy fluorescence images.
FIG. 24 shows the results of comparing quantitative distribution of transferrin receptor clusters formed in brain vessels, reticulocytes, lung, liver, and spleen, according to cluster size.
FIG. 25 shows the results of comparing average intensity values of all transferrin receptor clusters between brain vessels and reticulocytes.
FIG. 26 is a diagram schematically showing, as parameters for calculating the distance between transferrin receptors, the minimum value (min) and maximum value (max) of the distance between transferrin receptors.
FIG. 27 shows the results of comparing the density of total transferrin receptors between brain vessels and reticulocytes.
FIG. 28 shows the results of comparing the distance between transferrin receptors between brain vessels and reticulocytes.
FIG. 29 shows the results of confirming the thermodynamic structural stability of bonds between a transferrin receptor and helical region binding moiety #2, #3, #5, #6, #7, or #8 according to an embodiment.
FIG. 30 shows the results of confirming the thermodynamic structural stability of bonds between a transferrin receptor and a helical region binding moiety #9, #10, #11, #12, #13, or #14 according to an embodiment.
FIG. 31 shows the results of confirming the thermodynamic structural stability of bonds between a transferrin receptor and a helical region binding moiety #15, #16, #17, #18, #19, or #20 according to an embodiment.
FIG. 32 shows the results of confirming the thermodynamic structural stability of bonds between a transferrin receptor and a helical region binding moiety #21, #22, #23, or #24 according to an embodiment.
FIG. 33 shows the results of confirming the thermodynamic structural stability of bonds between a transferrin receptor and a helical region binding moiety #25, #27, #30, #32, #33, or #34 according to an embodiment.
FIG. 34 shows the results of confirming the thermodynamic structural stability of bonds between a transferrin receptor and a helical region binding moiety #36, #38, or#39 according to an embodiment.
FIG. 35 shows the results of confirming levels of intracellular delivery of a helical region binding moiety #04 according to an embodiment, by using a human brain endothelial cell line.
FIG. 36 shows the results of confirming levels of intracellular delivery of a helical region binding moiety #16, #19, or #20 according to an embodiment, by using a human brain endothelial cell line.
FIG. 37 shows the results of confirming levels of intracellular delivery of a helical region binding moiety #25, #26, or #27 according to an embodiment, by using a human brain endothelial cell line.
FIG. 38 shows the results of confirming levels of intracellular delivery of a helical region binding moiety #28, #29, or #31 according to an embodiment, by using a human brain endothelial cell line.
FIG. 39 shows the results of confirming levels of intracellular delivery of a helical region binding moiety #34, #35, #36, or #37 according to an embodiment, by using a human brain endothelial cell line.
FIG. 40 shows the results of confirming levels of intracellular delivery of a helical region binding moiety #38, #40, or #41 according to an embodiment, by using a human brain endothelial cell line.
FIG. 41 shows the results of confirming levels of IgG1 antibodies in brain tissue after intravenous administration of a blood-brain barrier permeable fusion protein F3#02, F3#03, F3#04, F3#05, or F3#06 according to an embodiment to an animal model, by using a human IgG1 ELISA kit.
FIG. 42 shows the results of confirming levels of IgG1 antibodies in brain tissue after intravenous administration of a blood-brain barrier permeable fusion protein F3#07, F3#08, F3#09, F3#10, or F3#11 according to an embodiment to an animal model, by using a human IgG1 ELISA kit.
FIG. 43 shows the results of confirming levels of IgG1 antibodies in brain tissue after intravenous administration of a blood-brain barrier permeable fusion protein F3#12, F3#13, F3#14, or F3#15 according to an embodiment to an animal model, by using a human IgG1 ELISA kit.
FIG. 44 shows the results of confirming levels of IgG1 antibodies in brain tissue after intravenous administration of a blood-brain barrier permeable fusion protein F3#16, F3#17, F3#18, F3#19, or F3#20 according to an embodiment to an animal model, by using a human IgG1 ELISA kit.
FIG. 45 shows the results of confirming levels of IgG1 antibodies in brain tissue after intravenous administration of a blood-brain barrier permeable fusion protein F3#21, F3#22, F3#23, or F3#24 according to an embodiment to an animal model, by using a human IgG1 ELISA kit.
FIG. 46 shows the results of confirming levels of IgG1 antibodies in brain tissue after intravenous administration of a blood-brain barrier permeable fusion protein F3#25, F3#26, or F3#27 according to an embodiment to an animal model, by using a human IgG1 ELISA kit.
FIG. 47 shows the results of levels of IgG1 antibodies in brain tissue after intravenous administration of a blood-brain barrier permeable fusion protein F3#28, F3#29, F3#30, F3#31, or F3#32 according to an embodiment to an animal model, by using a human IgG1 ELISA kit.
FIG. 48 shows the results of levels of IgG1 antibodies in brain tissue after intravenous administration of a blood-brain barrier permeable fusion protein F3#33, F3#34, F3#35, F3#36, or F3#37 according to an embodiment to an animal model, by using a human IgG1 ELISA kit.
FIG. 49 shows the results of confirming levels of IgG1 antibodies in brain tissue after intravenous administration of a blood-brain barrier permeable fusion protein F3#38, F3#39, F3#40, or F3#41 according to an embodiment to an animal model, by using a human IgG1 ELISA kit.
FIG. 50 shows the results of confirming levels of IgG1 antibodies in brain tissue after intravenous administration of a blood-brain barrier permeable fusion protein F1#03, F5#03, or F3#03 according to an embodiment to an animal model, by using a human IgG1 ELISA kit.
FIG. 51 shows the results of confirming levels of IgG1 antibodies in brain tissue after intravenous administration of a blood-brain barrier permeable fusion protein F1#05, F5#05, or F3#05 according to an embodiment to an animal model, by using a human IgG1 ELISA kit.
FIG. 52 shows the results of confirming levels of IgG1 antibodies in brain tissue after intravenous administration of a blood-brain barrier permeable fusion protein F1#06, F5#06, or F3#06 according to an embodiment to an animal model, by using a human IgG1 ELISA kit.
FIG. 53 shows the results of confirming levels of IgG1 antibodies in brain tissue after intravenous administration of a blood-brain barrier permeable fusion protein F1#12, F5#12, or F3#12 according to an embodiment to an animal model, by using a human IgG1 ELISA kit.
FIG. 54 shows the results of confirming levels of IgG1 antibodies in brain tissue after intravenous administration of a blood-brain barrier permeable fusion protein F1#16, F5#16, or F3#16 according to an embodiment to an animal model, by using a human IgG1 ELISA kit.
FIG. 55 shows the results of confirming levels of IgG1 antibodies in brain tissue after intravenous administration of a blood-brain barrier permeable fusion protein F1#25, F5#25, or F3#25 according to an embodiment to an animal model, by using a human IgG1 ELISA kit.
FIG. 56 shows the results of confirming levels of IgG1 antibodies in brain tissue after intravenous administration of a blood-brain barrier permeable fusion protein F1#27, F5#27, or F3#27 according to an embodiment to an animal model, by using a human IgG1 ELISA kit.
FIG. 57 shows the results of confirming levels of IgG1 antibodies in brain tissue after intravenous administration of a blood-brain barrier permeable fusion protein F1#31, F5#31, or F3#31 according to an embodiment to an animal model, by using a human IgG1 ELISA kit.
FIG. 58 shows the results of confirming levels of IgG1 antibodies in brain tissue after intravenous administration of a blood-brain barrier permeable fusion protein F1#37, F5#37, or F3#37 according to an embodiment to an animal model, by using a human IgG1 ELISA kit.
FIG. 59 shows the results of confirming levels of IgG1 antibodies in brain tissue after intravenous administration of a blood-brain barrier permeable fusion protein F1#40, F5#40, or F3#40 according to an embodiment to an animal model, by using a human IgG1 ELISA kit.
FIG. 60 shows the results of confirming changes in binding force of IgG1 antibodies to PD-L1 depending on the presence or absence of a binding moiety #01 in the blood-brain barrier permeable fusion proteins F3#01 and F3'#01 according to an embodiment.
FIG. 61 shows the results of confirming levels of IgG1 antibodies in ISF confirmed by using a human IgG1 ELISA kit after intravenous administration of a blood-brain barrier permeable fusion protein F3#25-Tau, which includes a Tau-specific IgG1 antibody, wherein the level was expressed as a multiple compared to a control group.
FIG. 62 shows the results of confirming levels of IgG1 antibodies in ISF confirmed by using a human IgG1 ELISA kit after intravenous administration of a blood-brain barrier permeable fusion protein F3#27-Tau or F3#36-Tau, which includes a Tau-specific IgG1 antibody, wherein the level was expressed as a multiple compared to a control group.
FIG. 63 shows the results of confirming levels of IgG1 antibodies in brain tissue confirmed by using a human IgG1 ELISA kit after intravenous administration of a blood-brain barrier permeable fusion protein F3#25-PD1, which includes a PD1-specific IgG1 antibody, wherein the level was expressed as a multiple compared to a control group.
FIG. 64 shows the results of confirming levels of IgG1 antibodies in brain tissue confirmed by using a human IgG1 ELISA kit after intravenous administration of blood-brain barrier permeable fusion protein F3#25-HER2, which includes a HER2-specific IgG1 antibody, wherein the level was expressed as a multiple compared to a control group.
FIG. 65 shows the results of confirming levels of IgG1 antibodies in brain tissue confirmed by using a human IgG1 ELISA kit after intravenous administration of a blood-brain barrier permeable fusion protein F3#25-Aβ, which includes an Aβ-specific IgG1 antibody, wherein the level was expressed as a multiple compared to a control group.

### Best Mode

### Mode for Invention

Hereinafter, preferable Examples are presented to help understanding of the present disclosure. However, Examples below are only presented for easier understanding of the present disclosure, and the contents of the present disclosure are not limited by the following examples.

### [Examples]

### Examples 1 to 41. Preparation of blood-brain barrier permeable fusion protein

In this example, a fusion protein with improved permeability to the BBB was to be prepared. For this purpose, a fusion protein in which the binding moiety to the helical region of the TfR was linked to the C-terminus regions of the light chain and the C-terminus regions of the heavy chain in an IgG1 antibody, that is, a total of four terminus regions was prepared. Meanwhile, in this example, as the binding moiety to the helical region of the TfR, a total of 24 binding moieties were derived, each having binding characteristics to the helical region but having a different amino acid sequence (first binding moiety group). Also, as the binding moiety to the helical region of the TfR, a total of 17 binding moieties were additionally derived, each maintaining a certain level of sequence identity with the binding moiety of SEQ ID NO: 3 and having substitution, insertion, or deletion of some of the amino acid sequences (second binding moiety group).

### 1. Preparation of fusion protein including tetravalent first binding moiety group

A BBB permeable fusion protein in which the binding moiety to the helical region of the TfR is linked to the aforementioned four terminus regions in the IgG1 antibody was prepared as follows. Specifically, 1 mL of sample was collected with a pipette from a flask containing cells, and the cell mass was measured therefrom. Afterwards, when the cell viability was 95% or more and the cell mass was 4 to 6X10⁶ cells/mL or more, a culture medium stored in an incubator at 37°C was added to the cells and cultured to prepare the cells such the cell mass level became 9X10⁶ cells/mL. Afterwards, transfection was performed on the prepared cells according to the following experimental conditions.

**[Table 1]**

| **200mL/ 1L** | **Transfection condition (based on 1 L flask)** | | | | | | |
|---|---|---|---|---|---|---|---|
| **Product Name** | **Cell mass (cells/m L)** | **DNA mixture** | | | **TF reagent mixture** | | **Reacti on Time** |
| | | **DNA(HC, ug)** | **DNA(LC,u g)** | **OptiPro( mL)** | **TF reage nt (mL)** | **OptiPr o (mL)** | **1 min<** |
| F3#01 | 9 ×10⁶ | 53.3 | 106.7 | 8.0 | 0.8 | 7.2 | 1 min< |

Afterwards, the feed media and enhancer were treated on Day 1 of culture, and the feed media was treated and cultured on Day 5 of culture. When the cell viability was 70% or less or 8 days after the transfection was performed, the transfected cells were obtained. Afterwards, the sample containing the transfected cells was centrifuged at 4500 rpm and 25°C for 15 minutes, and the supernatant was collected therefrom. Afterwards, the supernatant was filtered by using a 0.22 µm filter. Afterwards, the filtrate was purified by using purification techniques such as affinity chromatography and size exclusion chromatography, to obtain individual fusion proteins. The purified antibody was analyzed by size exclusion high performance liquid chromatography (SEC-HPLC), sodium dodecyl sulfate polyacrylamide gel electrophoresis (SDS-PAGE), and mass spectrometry, to confirm the purification results.

Meanwhile, in the fusion protein according to this example, details about the binding moiety to the helical region of the TfR, the IgG1 antibody, etc., are as follows.

### [Example 1]

| **Name** | **Composition** | **Detail** |
|---|---|---|
| F3#01 | Binding moiety | GHHERLKSDEWSVTSG (SEQ ID NO: 3) |
| | Valency of binding moiety | 4-valency |
| | IqG1 antibody | Anti-PD-L1 IqG1 antibody |
| | Binding site of IgG1 antibody | C-terminus of each of heavy chain and light chain |

### [Example 2]

| **Name** | **Composition** | **Detail** |
|---|---|---|
| F3#02 | Binding moiety | SREERLEEDRRRVDSG (SEQ ID NO: 4) |
| | Valency of binding moiety | 4-valency |
| | IgG1 antibody | Anti-PD-L1 IqG1 antibody |
| | Binding site of IgG1 antibody | C-terminus of each of heavy chain and light chain |

### [Example 3]

| **Name** | **Composition** | **Detail** |
|---|---|---|
| F3#03 | Binding moiety | TREAARRADEAEVDAG (SEQ ID NO: 5) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 4]

| **Name** | **Composition** | **Detail** |
|---|---|---|
| F3#04 | Binding moiety | GHDEKLKSDEKLVYSQ (SEQ ID NO: 6) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 5]

| **Name** | **Composition** | **Detail** |
|---|---|---|
| F3#05 | Binding moiety | SREERRLADEQEVLSG (SEQ ID NO: 7) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 6]

| **Name** | **Composition** | **Detail** |
|---|---|---|
| F3#06 | Binding moiety | SREAALAADEAAVESG (SEQ ID NO: 8) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 7]

| **Name** | **Composition** | **Detail** |
|---|---|---|
| F3#07 | Binding moiety | GLDEKLKSDETLVYSQ (SEQ ID NO: 9) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 8]

| **Name** | **Composition** | **Detail** |
|---|---|---|
| F3#08 | Binding moiety | SEEERRQEDEEEVERG (SEQ ID NO: 10) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 9]

| **Name** | **Composition** | **Detail** |
|---|---|---|
| F3#09 | Binding moiety | SHLERTKSDEWSIISEGL (SEQ ID NO: 11) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 10]

| **Name** | **Composition** | **Detail** |
|---|---|---|
| F3#10 | Binding moiety | SREERLREDERRVEEG (SEQ ID NO: 12) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 11]

| **Name** | **Composition** | **Detail** |
|---|---|---|
| F3#11 | Binding moiety | SREERLREDEEEVESG (SEQ ID NO: 13) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 12]

| **Name** | **Composition** | **Detail** |
|---|---|---|
| F3#12 | Binding moiety | SREERLEEDKQRVDSG (SEQ ID NO: 14) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 13]

| **Name** | **Composition** | **Detail** |
|---|---|---|
| F3#13 | Binding moiety | SREERLQQDEQEVDQG (SEQ ID NO: 15) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 14]

| **Name** | **Composition** | **Detail** |
|---|---|---|
| F3#14 | Binding moiety | SAEEERQRDREEVDNG (SEQ ID NO: 16) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 15]

| **Name** | **Composition** | **Detail** |
|---|---|---|
| F3#15 | Binding moiety | TAEEERQANEELVEAG (SEQ ID NO: 17) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 16]

| **Name** | **Composition** | **Detail** |
|---|---|---|
| F3#16 | Binding moiety | GLQEKLKSDEWSVLSQ (SEQ ID NO: 18) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 17]

| **Name** | **Composition** | **Detail** |
|---|---|---|
| F3#17 | Binding moiety | SREERRREDEREVEEG (SEQ ID NO: 19) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 18]

| **Name** | **Composition** | **Detail** |
|---|---|---|
| F3#18 | Binding moiety | SREERLREDEEEVDAG (SEQ ID NO: 20) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 19]

| **Name** | **Composition** | **Detail** |
|---|---|---|
| F3#19 | Binding moiety | GLQEKLKSDEKLVHSQ (SEQ ID NO: 21) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 20]

| **Name** | **Composition** | **Detail** |
|---|---|---|
| F3#20 | Binding moiety | GDEEKLKSDEELVDSQ (SEQ ID NO: 22) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 21]

| **Name** | **Composition** | **Detail** |
|---|---|---|
| F3#21 | Binding moiety | SREERRQADEEEVDSG (SEQ ID NO: 23) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 22]

| **Name** | **Composition** | **Detail** |
|---|---|---|
| F3#22 | Binding moiety | SEEEEREEDEEEVESG (SEQ ID NO: 24) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 23]

| **Name** | **Composition** | **Detail** |
|---|---|---|
| F3#23 | Binding moiety | GLDEKLKSDEKLVDSQ (SEQ ID NO: 25) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 24]

| **Name** | **Composition** | **Detail** |
|---|---|---|
| F3#24 | Binding moiety | GLDEKLKSDEDLVYSQ (SEQ ID NO: 26) |
| | Valency of binding moiety | 4-valency |
| | IgG1 antibody | Anti-PD-L1 IgG1 antibody |
| | Binding site of IgG1 antibody | C-terminus of each of heavy chain and light chain |

### 2. Preparation of fusion protein including tetravalent second binding moiety group

Fusion proteins including the second binding moiety group were prepared in the same manner as described above. Meanwhile, in the fusion proteins according to this example, details about the binding moiety to the helical region of the TfR, the IgG1 antibody, etc., are as follows.

### [Example 25]

| **Name** | **Composition** | **Detail** |
|---|---|---|
| F3#25 | Binding moiety | SHHERLKSDEWSVTSGGL (SEQ ID NO: 27) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 26]

| **Name** | **Composition** | **Detail** |
|---|---|---|
| F3#26 | Binding moiety | SHHERLKSDEWSVTSG (SEQ ID NO: 28) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 27]

| **Name** | **Composition** | **Detail** |
|---|---|---|
| F3#27 | Binding moiety | SHHERLKSDKWDVESGGL (SEQ ID NO: 29) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 28]

| **Name** | **Composition** | **Detail** |
|---|---|---|
| F3#28 | Binding moiety | LGHHERLKSDEWSVTSGGLIESESAET (SEQ ID NO: 30) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 29]

| **Name** | **Composition** | **Detail** |
|---|---|---|
| F3#29 | Binding moiety | ESKAVKWSALGHHERLKSDEWSVTSGGLIESESAET (SEQ ID NO: 31) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 30]

| **Name** | **Composition** | **Detail** |
|---|---|---|
| F3#30 | Binding moiety | SHHERLKSDEWNVTSR (SEQ ID NO: 32) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 31]

| **Name** | **Composition** | **Detail** |
|---|---|---|
| F3#31 | Binding moiety | HERLKSDEWSVKSG (SEQ ID NO: 33) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 32]

| **Name** | **Composition** | **Detail** |
|---|---|---|
| F3#32 | Binding moiety | GHHERLKSDEWSVT (SEQ ID NO: 34) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 33]

| **Name** | **Composition** | **Detail** |
|---|---|---|
| F3#33 | Binding moiety | SHHERLKSDEWSVTSW (SEQ ID NO: 35) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 34]

| **Name** | **Composition** | **Detail** |
|---|---|---|
| F3#34 | Binding moiety | SHHERLKADEWSVTSG (SEQ ID NO: 36) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 35]

| **Name** | **Composition** | **Detail** |
|---|---|---|
| F3#35 | Binding moiety | GHHERLKSDEWSVTSGGL (SEQ ID NO: 37) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 36]

| **Name** | **Composition** | **Detail** |
|---|---|---|
| F3#36 | Binding moiety | SHHERLKSDTWSVESGGL (SEQ ID NO: 38) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 37]

| **Name** | **Composition** | **Detail** |
|---|---|---|
| F3#37 | Binding moiety | ESKAVKWSALGHHEALKSDEWSVTSGGLIESESAET (SEQ ID NO: 39) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 38]

| **Name** | **Composition** | **Detail** |
|---|---|---|
| F3#38 | Binding moiety | GHHERLKSDFWSVTSG (SEQ ID NO: 40) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 39]

| **Name** | **Composition** | **Detail** |
|---|---|---|
| F3#39 | Binding moiety | GHHERLKSDEWS (SEQ ID NO: 41) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 40]

| **Name** | **Composition** | **Detail** |
|---|---|---|
| F3#40 | Binding moiety | ESKAVKWSALAHHERLKSDEWSVTSGGLIESESAET (SEQ ID NO: 42) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Example 41]

| **Name** | **Composition** | **Detail** |
|---|---|---|
| F3#41 | Binding moiety | GHHERLKSHQWEVESG (SEQ ID NO: 43) |
| | Valency of binding moiety | 4-valency |
| | IgG1 antibody | Anti-PD-L1 IgG1 antibody |
| | Binding site of IgG1 antibody | C-terminus of each of heavy chain and light chain |

### [Comparative Examples]

### Comparative Examples 1 to 22. Preparation of fusion protein linked with divalent binding moiety to the helical region of the TfR

A fusion protein in which two binding moieties to the helical region of the TfR were linked to either the C-terminus region of the heavy chain or the C-terminus region of the light chain in the IgG1 antibody (F1, F5) was prepared. Specifically, as the binding moiety to the helical region of the TfR, a total of six representative binding moieties (#01, #03, #05, #06, #12, and #16) among the first binding moiety group, and a total of 5 representative binding moieties (#25, #27, #31, #37, and #40) among the second binding moiety group were used.

### 1. Preparation of fusion protein including divalent first binding moiety group

A fusion protein was prepared in the same manner as described in Examples above, the fusion protein including: a binding moiety #01, #03, #05, #06, #12, or #16 as the binding moiety to the helical region of the TfR; anti-PD-L1 IgG1 antibody as the IgG1 antibody; and two binding moieties are linked to the C-terminus region of the heavy chain in the IgG1 antibody (Comparative Examples 1 to 6: F1#01, F1#03, F1#05, F1#06, F1#12, and F1#16).

In addition, a fusion protein was prepared in the same manner as described in Examples above, the fusion protein including: a binding moiety #01, #03, #05, #06, #12, or #16 as the binding moiety to the helical region of the TfR; anti-PD-L1 IgG1 antibody as the IgG1 antibody; and two binding moieties are linked to the C-terminus region of the light chain in the IgG1 antibody (Comparative Examples 7 to 12: F5#01, F5#03, F5#05, F5#06, F5#12, and F5#16).

### 2. Preparation of fusion protein including divalent second binding moiety group

A fusion protein was prepared in the same manner as described in Examples above, the fusion protein having a structure including: a binding moiety #25, #27, #31, #37, or #40 as the binding moiety to the helical region of the TfR; anti-PD-L1 IgG1 antibody as the IgG1 antibody; and two binding moieties linked to the C-terminus regions of the heavy chain in the IgG1 antibody (corresponding to Comparative Examples 13 to 17: F1#25, F1#27, F1#31, F1#37, and F1#40).

In addition, a fusion protein was prepared in the same manner as described in Examples above, the fusion protein having a structure including: a binding moiety #25, #27, #31, #37, or #40 as the binding moiety to the helical region of the TfR; anti-PD-L1 IgG1 antibody as the IgG1 antibody; and two binding moieties linked to the C-terminus regions of the light chain in the IgG1 antibody (corresponding to Comparative Examples 18 to 22: F5#25, F5#27, F5#31, F5#37, and F5#40).

### [Experimental Examples]

### Experimental Example 1. Evaluation of functionality of BBB permeable fusion protein F3#01

### 1-1. Evaluation of functionality of binding moiety

In this experimental example, the functionality of the binding moiety #01 of the BBB permeable fusion protein F3#01 according to an embodiment was to be determined by binding ability to the helical region of the TfR and by evaluating the level of delivery into human brain endothelial cells through the binding.

### (1) Confirmation of binding between helical region of TfR and binding moiety

In the prepared fusion protein F3#01, the binding between the helical region binding moiety and the TfR was confirmed by cryo-electron microscopy (Cryo-EM). Specifically, to prepare a vitrified frozen specimen, a 10 uM L7 TfR sample specimen was prepared by using 50 mM Tris, 150 mM NaCl, and a pH 7.6 buffer solution. Afterwards, vitrification of the L7 TfR sample specimen was performed by using a Quantifoil Cu 1.2/1.3 400 mesh grid. Here, in order to improve the interaction between the sample specimen and the grid, a glow discharger was used to induce negative charge discharge on the grid surface. Afterwards, 3 µl of the L7 TfR sample specimen was injected onto the discharged grid, and vitrification was performed thereon under the conditions of blotting time (7 seconds), blotting force (0), waiting time (0 second), 4°C, and 95% humidity. Also, the Cryo-EM analysis was performed by using 200 kV Glycios, 300 kV Krios G4, and Falcon 4 and K3 detectors. Here, the lens was set to the following conditions: spherical aberration rate (2.7), 0 50 uM aperture, magnification (120 K), and exposure time (6.55 seconds). In addition, the analysis was conducted under the conditions of 50 fractions per sample, 225 frames, pixel value of 0.894 Å/pix, dose rate of 6.1 e/px/s, total dose of 49.93 e/Å², and the defocus range within a range of -1.25 to -2.75 through an interval of 0.25. A total of at least 5 million particles were picked first, and then used for initial 2D classification. Afterwards, approximately 2 million particles were finally selected and subjected to 3D refinement, and C1 symmetry and C2 symmetry were each applied to derive a 3D electron density map with a resolution of about 3.4 Å. In the map, the co-complex model (PDB: 3s9n) of the TfR was overlaid, and the binding site and key residues were predicted by comparing the existing binding method and the binding method of the helical region binding moiety.

Also, the binding between the TfR and the helical region binding moiety #01 was confirmed through docking simulation. Specifically, the docking simulation was carried out to confirm whether the binding moiety #01 had binding ability to the helical region of the TfR. The structure of the binding moiety #01 was modeled by using a RosettaRelax program, and the position expected to interact with the helical region of the TfR was modeled by using structural information and thermodynamic calculation. Afterwards, the docking simulation that can find the most stable position by randomly changing the position of the binding moiety and calculating the interaction with the helical region was carried out by using a RosettaDocking program. 20,000 simulations were carried out for each sequence ID number, and the resulting data were analyzed based on homology to the initial modeling structure and the thermodynamic structural stability.

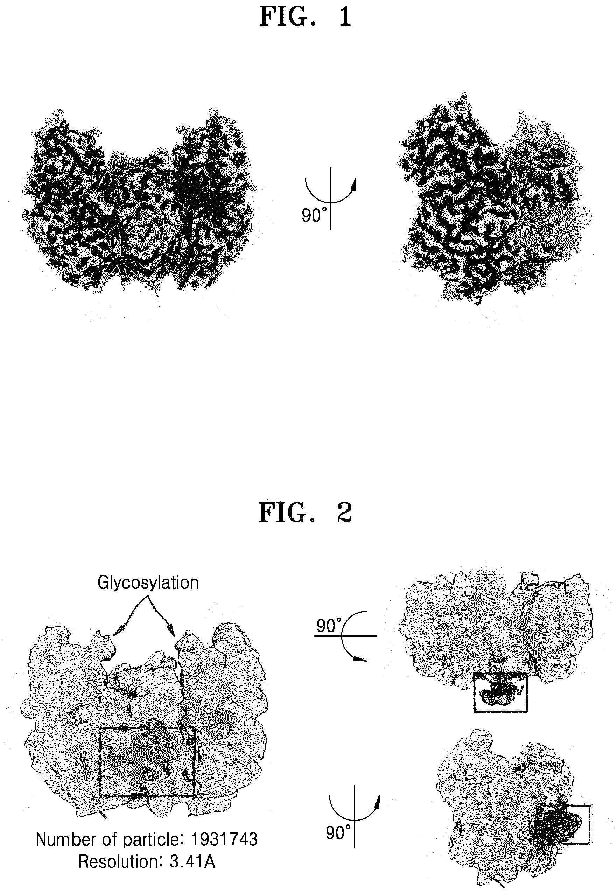

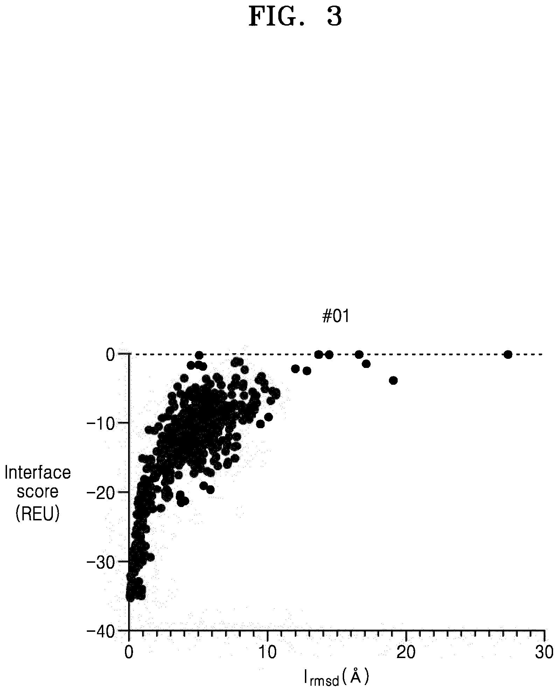

As a result, as shown in FIGS. 1 and 2, it was confirmed that the helical region available for the interaction with the TfR was exposed to the outside. Also, by fitting a model, which maintains the endogenous binding mode of transferrin, to the TfR in the 3D electron density map with a resolution of 3.4 Å, it was confirmed that the electron density equivalent to glycosylation, which has not been seen in the existing TfRs, existed in the ideal ranged predicted. Also, the binding with the binding moiety was formed to match the shape of the alpha helix structure, and through this, the interaction between the helical region of the TfR and the binding moiety was confirmed. Furthermore, as shown in FIG. 3, it was confirmed that the binding moiety #01 corresponding to SEQ ID NO: 3 stably binds to the helical region of the TfR.

### (2) Confirmation of delivery into human brain endothelial cells

It was aimed to determine whether the helical region binding moiety #01, which was confirmed to bind to the TfR in Section (1) of Experimental Example 1-1, would be able to be delivered into hCMEC/D3 cells, which are human brain endothelial cells constituting the human BBB, through the aforementioned interaction. Specifically, the hCMEC/D3 cells were cultured under conditions of 37 °C and 5 % CO₂ by using a synthetic culture solution, endothelial cell basal medium 2 (EBM2), containing growth factors. Afterwards, when the cell saturation reached 80%, the cells were separated, and 4X10³ cells were added to 40 µL of culture medium. Then, the cells were inoculated on a 384-well plate, centrifuged for 10 seconds, and cultured for at least 18 hours under conditions of 37°C and 5% CO₂ so that the cells were allowed to attach to the plate. Meanwhile, 500 µL of 1x PBS was added to and mixed with 1 mg of a binding moiety peptide, and the concentration of the mixture was measured by using an ultraviolet-visible spectrometer. The peptide was diluted to have a final concentration of 200 µm, and then stored at 4°C. Afterwards, the peptide at the concentration of 200 µm was diluted in an EBM2 culture solution at a concentration 5 times the treatment concentration. 10 µL of the peptide diluted 5-fold was inoculated on each well, centrifuged at 1,000 rpm for 10 seconds, and cultured for 2 hours under conditions of a temperature of 37°C and 5% CO₂. Afterwards, the cultured cells were washed with 1x PBS, and 75 µL of 4% PFA was inoculated on each well and stored at room temperature for 30 minutes for immobilization. Here, a Hoechst solution was added to the 4% PFA to simultaneously proceed the nuclear staining, and 30 minutes later, the cells were imaged by using a Cytation5 (Biotek) to evaluate the level of delivery into the cells.

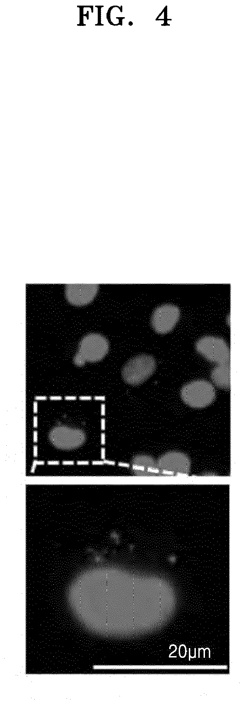

As a result, as shown in FIG. 4, the effective delivery ability of the binding moiety #01 corresponding to SEQ ID NO: 3 into the cells was confirmed as a result of evaluating the level of delivery into the cells by using the human brain endothelial cell line (hCMEC/D3).

### 1-2. Evaluation of level of BBB permeability

In this experimental example, the level of uptake of IgG1 antibodies into the brain tissue by intravenous administration of the BBB permeable fusion protein, F3#01, according to an embodiment was evaluated.

### (1) Evaluation of IgG1 antibody levels in brain tissue over time

A preparation containing 20 mg/kg of the fusion protein F3#01 was intravenously administered to a C57BL/6 mouse through the caudal vein, and a group intravenously administered with the IgG1 antibody only was used as a control group. After 1, 4, 7, or 14 days, the mouse was anesthetized and blood was collected from the blood vessels inside the eyes or from the abdominal vein. Afterwards, the blood was removed by perfusion with physiological saline. Subsequently, the brain of the mouse was extracted, and the extracted brain tissue was rapidly frozen with liquid nitrogen and stored in a deep freezer until use. Meanwhile, the extracted brain tissue was homogenized by using a protein extraction solution, and a sample of the homogenized brain tissue was then dissolved at 4°C by using a rotating mixer. Afterwards, the dissolved sample of brain tissue was centrifuged, and the supernatant was obtained therefrom to prepare a brain lysate. Afterwards, the level of IgG1 antibodies in the brain lysate was measured by using a human IgG1 ELISA kit. Specifically, a standard (STD) was prepared by dilution to each concentration by using a protein extraction solution, and a sample (SPL) containing the brain lysate was prepared according to each dilution ratio by using a protein extraction solution. Then, 50 uL of each of the two was added to each well. Afterwards, 50 uL of an Ab cocktail contained in an ELISA kit was additionally added to each well, and then a reaction was allowed at 4°C. Afterwards, the plate was washed, and 100 uL of a TMB substrate was added to each well of the washed plate. Subsequently, using a microplate reader, a stop solution was added when the OD value of STD1 reached 1.0 at a wavelength of 600 nm. After the value at a wavelength of 450 nm was measured, a standard curve was obtained by using 4 parameter logistic regression, and therefrom, the concentration of IgG1 antibodies present in the sample (SPL) was calculated.

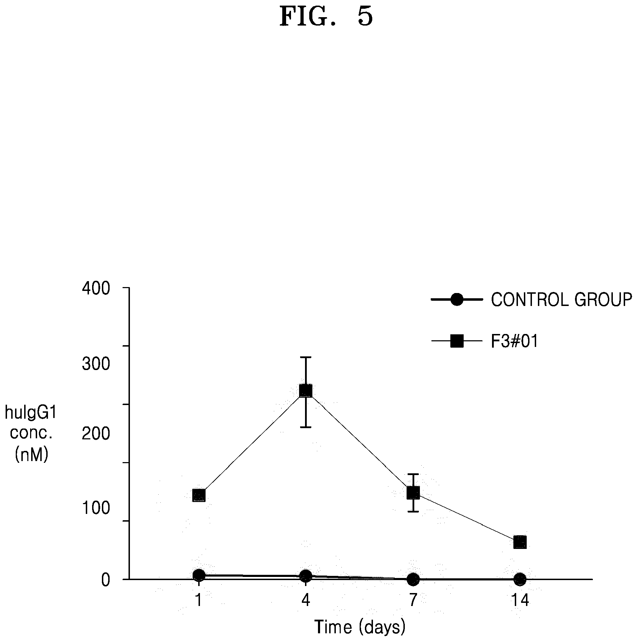

As a result, as shown in FIG. 5, the level of IgG1 antibodies in the brain tissue of the F3#01 administration group according to an embodiment was observed to be significantly higher than that of the control group until 14 days after the administration. Specifically, the F3#01 administration group showed a high level of delivery ranging from about 25-fold to about 320-fold than the control group.

### (2) Evaluation of IgG antibody levels in brain tissue by using ISF sample

After the fusion protein penetrated the BBB, the amount of the fusion protein (fusion protein present in an ISF sample) present in an unbound form in the brain parenchyma was detected, followed by recovery for the detected value. Accordingly, the amount of the fusion protein that entered the brain parenchyma from the brain vessels, i.e., the amount of the fusion protein that penetrated the BBB, was evaluated. Specifically, after anesthetizing a C57BL/6 mouse, the skin on the head of the mouse was incised, and a drill was used to form a perforation in the cranial bone adjacent to the hippocampus area. Afterwards, a guide cannula was inserted into the formed cranial perforation, and then fixed by using resin. The skin incisions were sutured to prevent the cranial perforation area from being exposed to the outside, and the mouse was allowed to recover for two weeks. Thereafter, a preparation containing 20 mg/kg of the fusion protein (F3#01) was intravenously administered to the recovered mouse through the caudal vein.

At 4 hours, 1 day, or 4 days after the intravenous administration of the fusion protein to the mouse, the mouse was anesthetized, and a probe activated for detecting IgG1 antibodies was inserted into the probe guide cannula of the mouse. Thereafter, while flowing BSA-containing cerebrospinal fluid (CSF) at a constant rate, a sample of interstitial fluid (ISF) containing the fusion protein was collected and stored at -20°C. Afterwards, the level of IgG1 antibodies in the ISF sample was measured by using a human IgG1 ELISA kit. Specifically, a standard (STD) and a sample (SPL) that were diluted to each concentration by using an N.S sample buffer were prepared, and then 50 uL of each of the two was added to each well. Afterwards, 50 uL of an Ab cocktail contained in an ELISA kit was additionally added to each well, followed by incubation at 4°C. Afterwards, the plate was washed, and 100 uL of a TMB substrate was added to each well of the washed plate. Subsequently, using a microplate reader, a stop solution was added when the OD value of STD1 reached 1.0 at a wavelength of 600 nm, and the value at a wavelength of 450 nm was measured. Meanwhile, a group intravenously administered with the IgG1 antibody only was used as a control group.

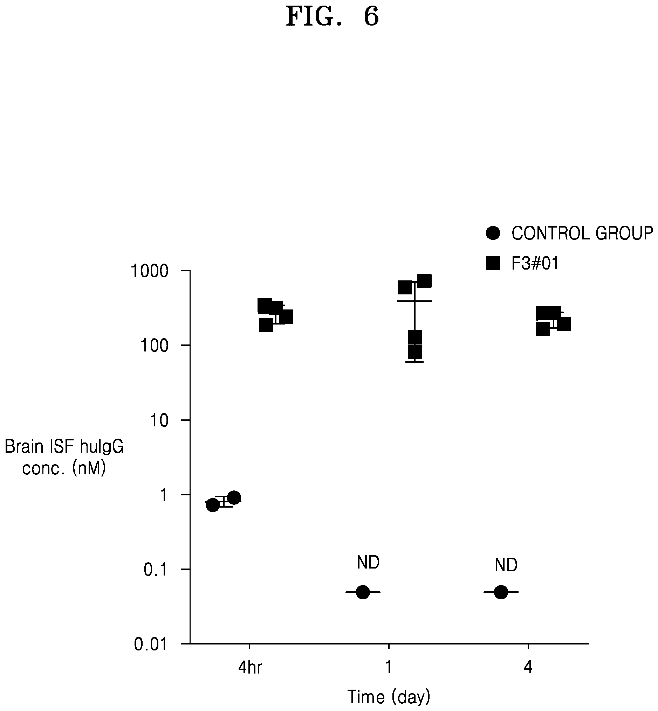

As a result, as shown in FIG. 6, it was confirmed that the level of IgG1 antibodies in the ISF sample was significantly increased in the F3#01 administration group according to an embodiment compared to the control group.

### (3) Evaluation of IgG antibody levels through brain vessel imaging in animal model

By using an animal model implanted with a cranial window and a two-photon microscope, real-time images of the cerebrovascular region were aimed to be acquired to confirm a transcytosis phenomenon of the fusion protein in the BBB. Specifically, a Tie2-GFP Tg mouse implanted with a cranial window was used, and structural images were acquired through a two-photon microscope from the region where the pial blood vessels originate to a depth of about 300 µm, including 2 to 3 cortical layers. Afterwards, to observe transcytosis of Alexa-568-bound fusion protein, 26 mg/kg of Alexa-568-bound fusion protein was injected through the caudal vein. In the same 3D area as the image acquired 1 day after the injection of the fusion protein, the fluorescence intensity of GFP was measured in the first channel and the fluorescence intensity of Alexa-568 bound to the fusion protein was measured in the second channel. The measurement of the fluorescence intensity was performed for 20 minutes under conditions with a spatial resolution of 100 nm or less and a temporal resolution of less than a minute, in an area including at least 3 segments of blood vessel beginning from the postcapillary venule. To observe the transcytosis phenomenon, all acquired time series images were aligned to the first time series image, aligned to coordinates of the first time series image, and then divided into intravascular, vessel wall, and extravascular areas by the first channel GFP signals. Among signals of the Alexa-568 bound to the fusion protein observed in the second channel based on the intravascular, vessel wall, and extravascular areas divided by the first channel GFP signals, the area clustered near the vessel wall was redesignated as an intravascular region (ROI 1; region of interest); a vessel wall region (ROI 2), and an extravascular region (ROI 3), and changes in the concentration of the Alexa-568 bound to the fusion protein in the three ROIs were observed over time.

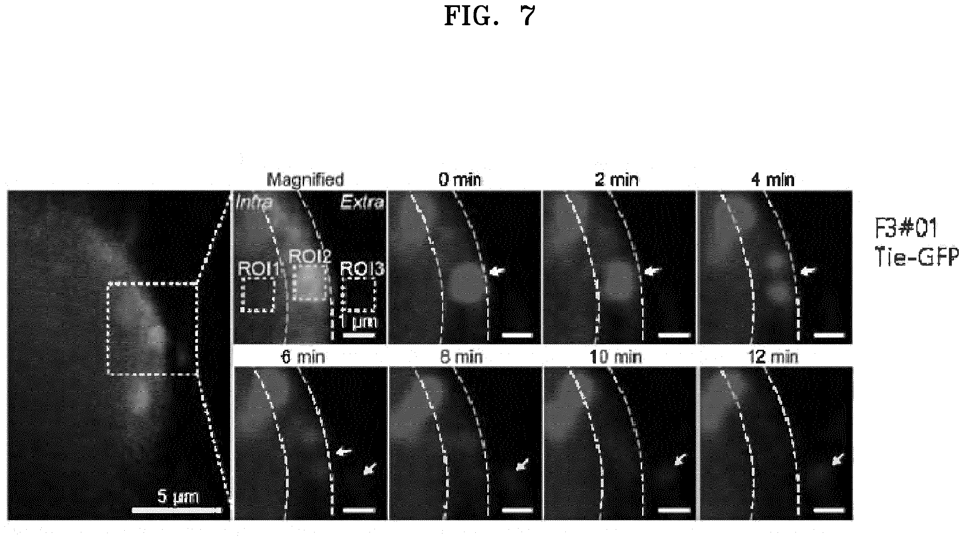

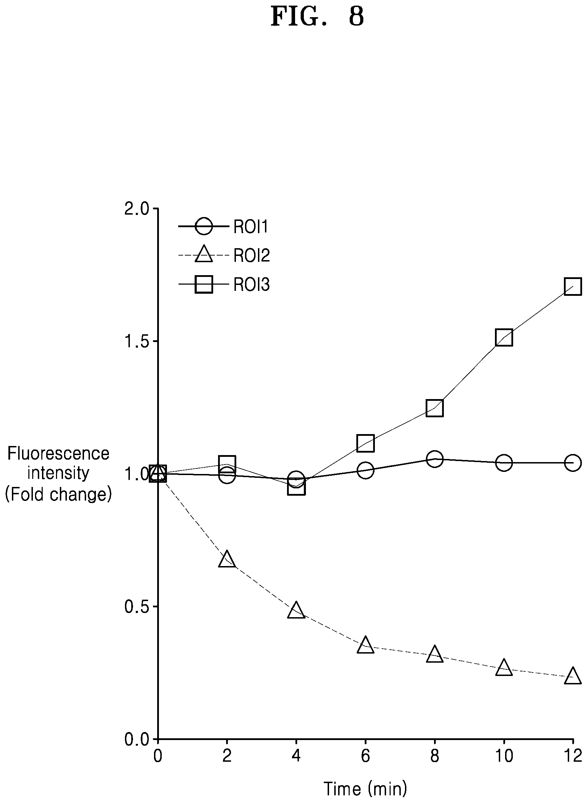

As a result of observing real-time transcytosis changes in three ROIs for 20 minutes on the first day after the injection of the fusion protein according to an embodiment, as shown in FIGS. 7 and 8, it was confirmed that no change in the concentration of the fusion protein in the intravascular region ROI 1 was observed, whereas, in the vessel wall region ROI2 where clusters exist, the fusion protein clustered for 10 minutes and migrated to the outside of the blood vessel so that the concentration of the fusion protein decreased. Then, from the moment when no more clusters were observed (>10 minutes), the concentration of the fusion protein was observed to be in an equilibrium state. Also, regarding the change in the concentration of the fusion protein in the extravascular region ROI 3, it was observed that the fusion protein clusters initially observed in the vessel wall region ROI 2 reached the area outside the blood vessel after about 6 minutes, and at the same time, that the concentration of the fusion protein increased. Afterwards, the concentration was the observed to peak at about 12 minutes. The results above indicate that transcytosis of the fusion protein occurred through clustering near the BBB, followed by extravasation of the fusion protein within a few minutes.

### 1-3. Evaluation of functionality based on change in valency of binding moiety

20 mg/kg of each of the fusion proteins F3#01, F1#01, and F5#01 was formulated into a C57BL/6 mouse and administered intravenously through the caudal vein. At 4 days thereafter, the level of IgG1 antibodies in brain tissue were measured in the same manner as in Section (1) of Experimental Example 1-2. Afterwards, a standard curve was obtained by using 4 parameter logistic repression, and therefrom, and the concentration of IgG1 antibodies present in the sample (SPL) was calculated. Furthermore, in order to observe the state of antibody delivery inside the brain vessels, the brain of an animal was extracted 1 day after the intravenous administration of the fusion protein. After anesthetizing the animal, the thoracic cavity of the animal was opened, and a butterfly needle was inserted into the left ventricle to inject a saline solution and drain it into the right atrium to remove blood from the body. Afterwards, 4% paraformaldehyde (PFA) was injected to fix the cells, and the cranial bond was incised to extract the brain. The extracted brain was stored in 4% PFA for one day for additional fixation of the cells, and then stored in a 30% sucrose solution for 3 days to prevent cell destruction during preparation of brain slices. The mouse brain stored in the 30% sucrose solution for 3 days was placed in a mold for brain slice preparation, an optimal cutting temperature compound solution was injected thereto, and the brain was stored at -60°C to cool the brain. A sample of the cooled brain was cut into 40-pm-thick brain slices by using a micro-cryostat, and each brain slice was used for immunostaining. Afterwards, immunostaining was performed by using a TfR antibody and an antibody against the fusion protein, and the results were photographed and confirmed with a confocal microscope.

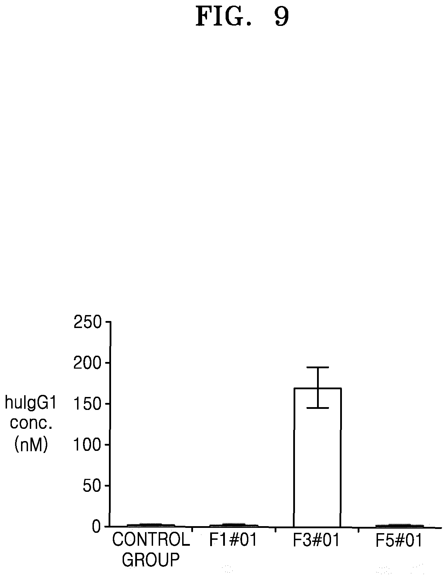

As a result, as shown in FIG. 9, at 4 days after intravenous administration, the level of IgG1 antibodies in the brain tissue was quantified as a relative value to the control group, and compared. As a result, it was confirmed that, compared to the control group, the group administered with a tetravalent fusion protein in which the binding moieties were linked to each of four regions including the heavy chain C-termini and the light chain C-termini, i.e., the F3#01 administration group, showed an about 80-fold increase in the level of IgG1 antibodies in the brain tissue. On the other hand, the groups administered with a divalent fusion protein in which the binding moieties were linked to two regions including the heavy chain C-termini or the light chain C-termini, i.e., the F1#01 administration group and the F5#01 administration group, showed no significant difference in the level of IgG1 antibodies from the control group despite using the same binding moiety.

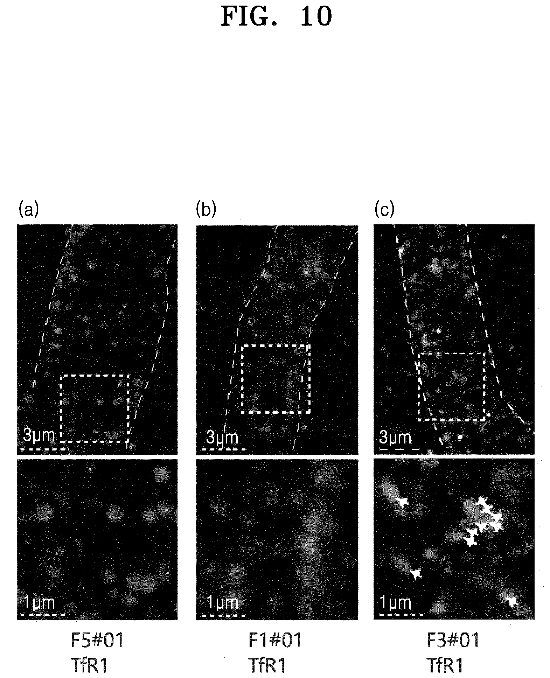

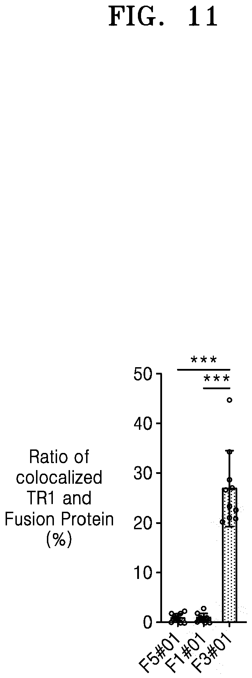

In addition, as shown in FIGS. 10 and 11, no binding between the fusion protein and the TfR was observed in the brain vessels of the F1#01 administration group and the F5#01 administration group, whereas binding between the fusion protein and the TfR was observed in the brain vessels of the F3#01 administration group. As a result of quantifying these results, the level of the fusion protein present in the region where the TfR was located showed a significant difference.

### 1-4. Evaluation of interaction between fusion protein and TfR receptor

Under the premise that the high permeability to the BBB confirmed in Experimental Example 1-2 is due to the interaction between the binding moiety and the TfR present in brain tissue, negative staining transmission electron microscope (TEM) analysis was performed to confirm the interaction between the fusion protein, i.e., the tetravalent binding moiety, and the TfR. Specifically, samples containing the fusion protein and the TfR added together were prepared as experimental groups, and as control groups, (1) a group in which only a native TfR was added, (2) a group in which only the IgG1 antibody without the binding moiety was added, (3) a group in which the TfR and the IgG1 antibody without the binding moiety were added together without the binding moiety were prepared. All samples were quantified at 15 uM by using a buffer solution of 50 mM Tris, 150 mM NaCl, and pH 7.4, and the group in which the fusion protein and the TfR were added together and the group in which the IgG1 antibody and the TfR were added together were mixed at a certain molar ratio (1:2) to proceed a reaction at 4°C for 1 hour. Afterwards, all samples were diluted to an appropriate concentration, and TEM grid sampling was performed thereon. The grid for the electron microscope specimen was a 400 mesh F/C Cu grid, and 10 ul of each the samples prepared as described above was injected onto the grid and allowed for a reaction for 1 minute, and the grid was then removed by using a filter paper. Afterwards, 10 ul of a 2% uranyl acetate solution was injected onto the grid, and the remaining solution was removed by using a filter pater again. The grid was also exposed to room temperature conditions for about 12 hours to remove any remaining moisture. The grid specimens prepared as described above were observed by using a 60kV Jeol Gatan TEM, and negative staining sample analysis was performed at 60 K to 200 K magnification.

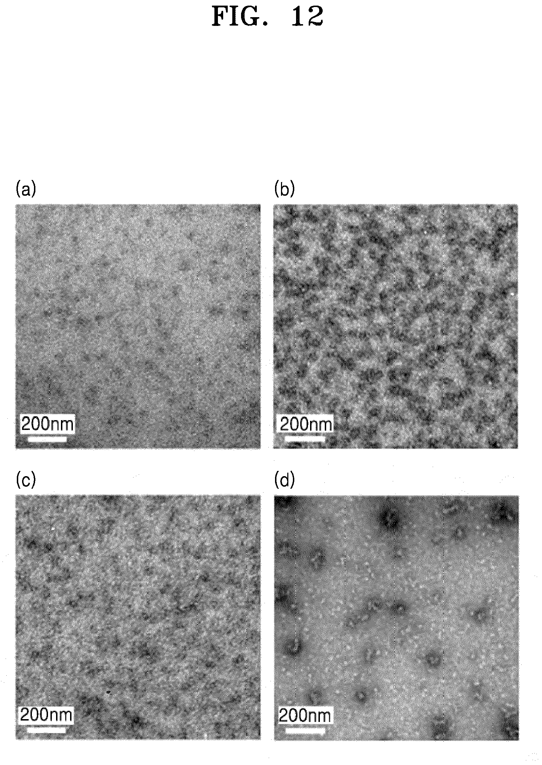

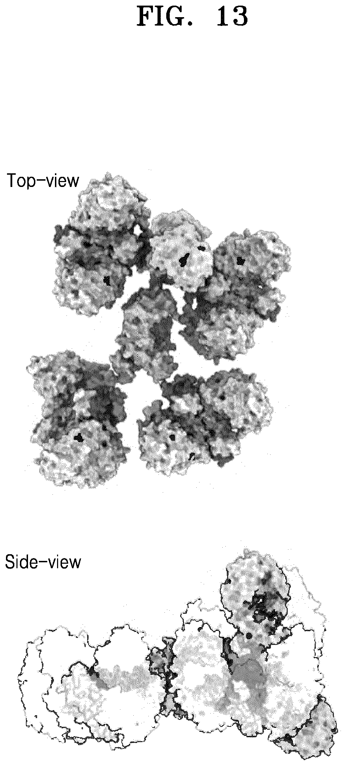

As a result, as shown in FIG. 12, the group in which the TfR and the IgG1 antibody without the binding moiety were added together ((c) in FIG. 12), as well as the group in which only the native TfR was added and the group in which the IgG1 antibody without the binding moiety was added ((a) and (b) in FIG. 12), showed a relatively homogeneous distribution of each component, confirming the absence of interactions therebetween. On the other hand, the group in which the fusion protein according to an embodiment and the native TfR were added together ((d) of FIG. 12) showed the formation of a complex through interactions with the TfR. Furthermore, based on these experimental results, it was found that each of the tetravalent binding moieties according to an embodiment interacts with the helical region of the TfR to form a complex, as shown in FIG. 13. In particular, considering the experimental results in Experimental Examples 1 to 3 (see FIG. 9), the experimental results of this Example indicate that the interactions within the complexes may be a major factor in improving the permeability to the BBB.

### 1-5. Evaluation of selective delivery efficacy to brain tissue

In this experimental example, the organ-specific distribution level of IgG1 antibodies by intravenous administration of the BBB permeable fusion protein, F3#01, according to an embodiment was evaluated. Specifically, a preparation containing 20 mg/kg of the fusion protein F3#01 was intravenously administered to a C57BL/6 mouse through the caudal vein, and a group intravenously administered with the IgG1 antibody only was used as a control group. Then, 1 or 4 days after the intravenous administration, the level of IgG1 antibodies in a total of 6 organs (brain, lung, spleen, kidney, liver, and muscle) was measured in the same manner as described in Section (1) of Experimental Example 1-2, and compared.

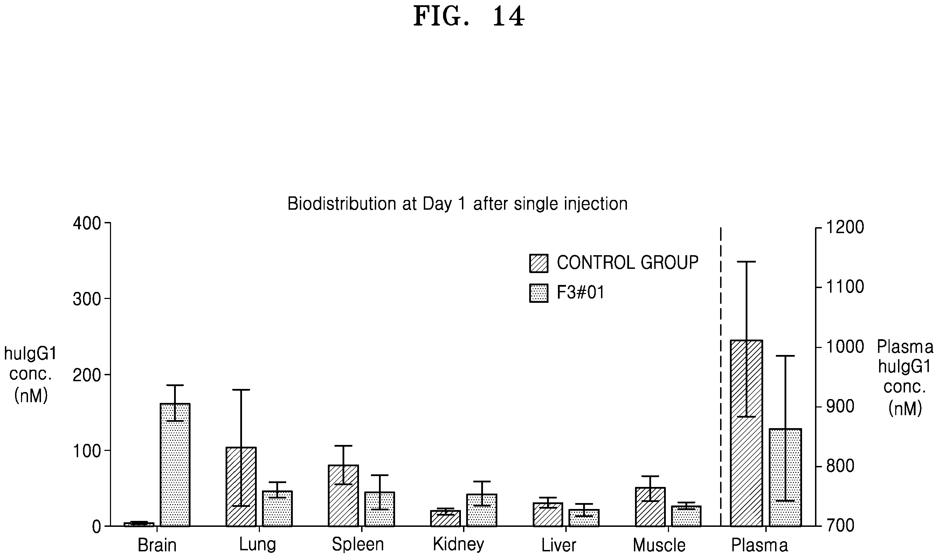

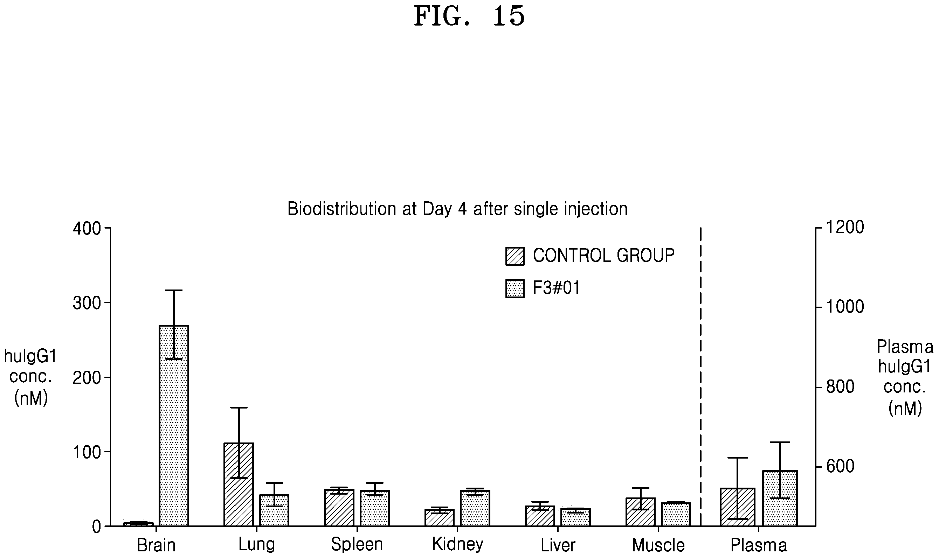

As a result, at 1 day after the intravenous administration of the BBB permeable fusion protein, F3#01, the organ-specific distribution level of the IgG1 antibody is as shown in FIG. 14, and at 4 days after the intravenous administration of the BBB permeable fusion protein, F3#01, the organ-specific distribution level of the IgG1 antibody is as shown in FIG. 15. In other words, the control group in which the IgG1 antibody was intravenously administered showed the highest distribution level of the IgG1 antibody in lungs among the organs, and a relatively low distribution level in the brain tissue, whereas the F3#01 administration group was confirmed to have a very high level of the IgG1 antibody in the brain tissue compared to other organs. Referring to the results above, it was confirmed that the fusion protein containing the functional structure according to an embodiment can contribute to reducing the side effects of antibody-based drugs by reducing the distribution of IgG antibodies in tissues other than the brain tissue, due to selective distribution of the IgG antibodies in the brain tissue upon the intravenous administration.

### 1-6. Evaluation of reduction of side effects by selective delivery to brain tissue

### (1) Evaluation of reticulocyte levels

After administering the BBB permeable fusion protein, F3#01, according to an embodiment, it was aimed to determine effects on reticulocytes which are one of cells in which the TfR is highly expressed. In detail, a preparation containing 20 mg/kg or 50 mg/kg of the fusion protein F3#01 was intravenously administered to a C57BL/6 mice through the caudal vein. At 0, 1, 4, or 7 days after the intravenous administration of the fusion protein to the mouse, 50 µl of a blood sample was obtained from the blood vessel inside the eye of the mouse by using a heparinized capillary tube. Afterwards, 30 µl of the blood sample and 100 µl of a new methylene blue solution were mixed, and the mixed solution was left at room temperature for a certain period of time. Afterwards, the stained blood sample was smeared on a glass slide and observed under a microscope to calculate the percentage of reticulocytes among all red blood cells. Meanwhile, a group intravenously administered with the IgG1 antibody was used as a control group.

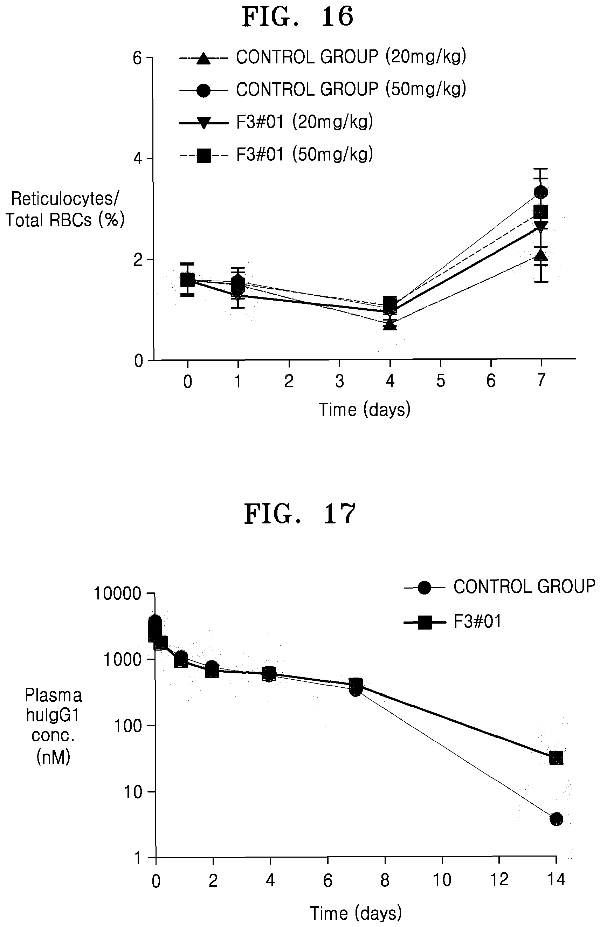

As a result, as shown in FIG. 16, in the group administered with the BBB permeable fusion protein, F3#01, according to an embodiment, the percentage of reticulocytes among total red blood cells was similar to that of the control group.

### (2) Pharmacokinetic evaluation

After administering the BBB permeable fusion protein, F3#01, according to an embodiment to a mouse, the pharmacokinetic evaluation in plasma was performed. In detail, a preparation containing 20 mg/kg of the fusion protein F3#01 was intravenously administered to a mouse through the caudal vein. Blood samples were obtained from the mouse 30 minutes, 120 minutes, 360 minutes, 1 day, 2 days, 4 days, 7 days, or 14 days after the intravenous administration of the fusion protein to the mouse. Afterwards, the plasma samples were separated by centrifugation, pre-treated for liquid chromatography mass spectrometry (LC-MS), and then subjected to LC-MS analysis. Meanwhile, a group intravenously administered with the IgG1 antibody was used as a control group.

As a result, as shown in FIG. 17, the plasma PK profile of the group administered with the BBB permeable fusion protein, F3#01, according to an embodiment was similar to that of the control group, and thus the experimental results above indicate that the distribution of the fusion protein in several organs including the TfR was minimal.

### (3) Calculation of blood-to-plasma ratio

To verify that the experimental results above result from the selective delivery to brain tissue, i.e., non-binding with reticulocytes including the TfR and with other organ tissues, a blood-to-plasma ratio (peak area ratio of blood supernatant / peak area ratio of plasma) was calculated. In detail, the BBB permeable fusion protein, F3#01, according to an embodiment was added to plasma and a blood sample to have a final concentration of 40 µg/mL, and then left at room temperature for 30 minutes. Afterwards, the blood sample was centrifuged, and the blood supernatant was separately obtained. The plasma and blood supernatant samples were mixed with a PBS solution containing a surfactant, and magnetic beads, the mixture was cultured, and the culture was washed twice with PBS containing a surfactant. Here, RapiGest surfactant and dithiothreitol were added, incubated at 60°C for 50 minutes, and left at room temperature for 10 minutes. Afterwards, 1) iodoacetic acid was added and cultured in the dark at room temperature for 30 minutes, 2) trypsin was added and cultured at 60°C for 24 hours, sequentially, 3) HCl was added and cultured at 37°C for 30 minutes. Afterwards, the culture was centrifuged to obtain the supernatant, trypsin was added to the supernatant, and a blood-to-plasma ratio (peak area ratio of blood supernatant / peak area ratio of plasma) was calculated by using LC-MS. Meanwhile, a group treated with the IgG1 antibody was used as a control group.

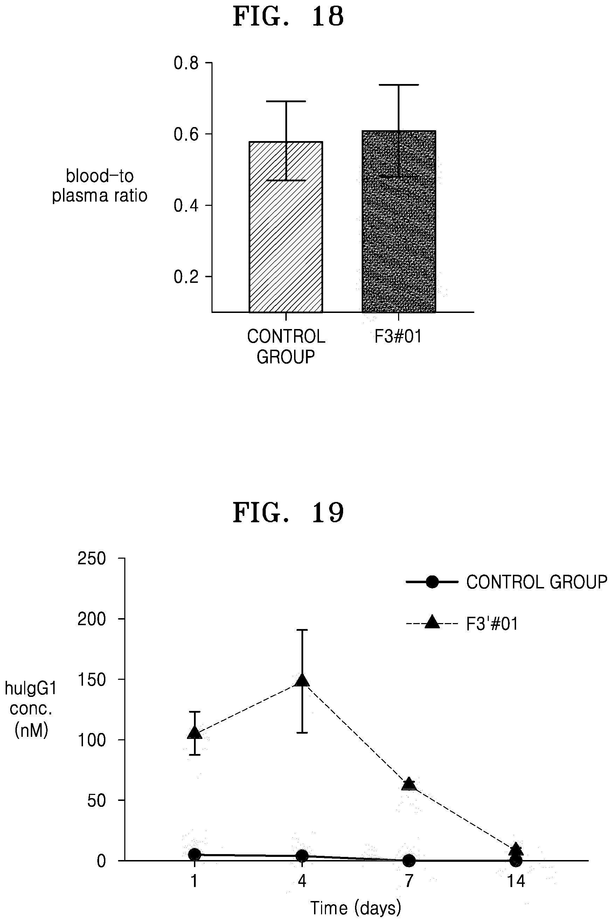

As a result, as shown in FIG. 18, the blood-to-plasma ratio of the group administered with the BBB permeable fusion protein, F3#01, according to one embodiment also showed a similar level to that of the control group, and thus it was confirmed that the experimental results above result from the fact that no binding was formed between the fusion protein and the reticulocytes including the TfR. Also, the selective delivery to brain tissue was confirmed by referring that the BBB permeable fusion protein according to an embodiment did not show high levels of TfR-mediated delivery in organs other than the brain tissue.

### 1-7. Evaluation of functionality of fusion protein including repeated binding moieties

In this experimental example, it was aimed to determine whether the functionality of the aforementioned fusion protein could be maintained, even when the binding moiety to the helical region of the TfR is modified to repeat. For this purpose, in the same manner as in Example above, a fusion protein (F3'#01) having a structure in which, as the binding moiety to the helical region of the TfR, a binding moiety in which a moiety of SEQ ID NO: 3 is repeated twice was used, an anti-PD-L1 IgG1 antibody was used as the IgG1 antibody; and a total of four binding moieties are linked to the C-termini of each of the heavy and light chains in the IgG1 antibody.

### (1) Evaluation of IgG1 antibody levels in brain tissue over time

The level of uptake of the IgG1 antibody into brain tissue by intravenous administration of the prepared BBB permeable fusion protein, F3'#01, according to an embodiment was evaluated in the same manner as in Section (1) of Experimental Example 1-2.

As a result, as shown in FIG. 19, the level of IgG1 antibodies in the brain tissue of the F3'#01 administration group according to an embodiment was observed to be higher than that of the control group until 7 days after the administration. Specifically, the F3'#01 administration group showed a high level of delivery ranging from about 18-fold to about 160-fold than the control group.

### (2) Evaluation of selective delivery efficacy to brain tissue

The organ-specific distribution level of the IgG1 antibody by intravenous administration of the prepared BBB permeable fusion protein, F3'#01, according to an embodiment was evaluated in the same manner as in Experimental Example 1-5.

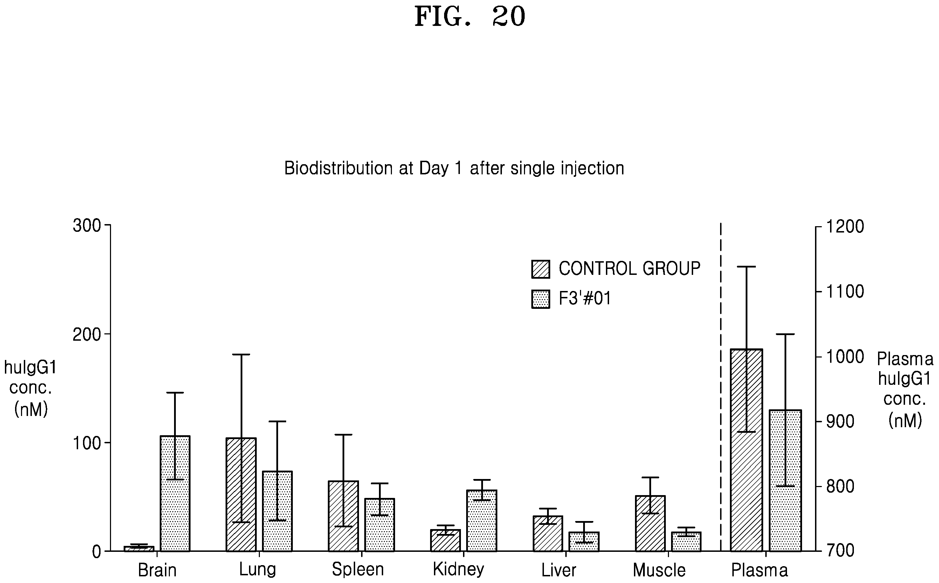

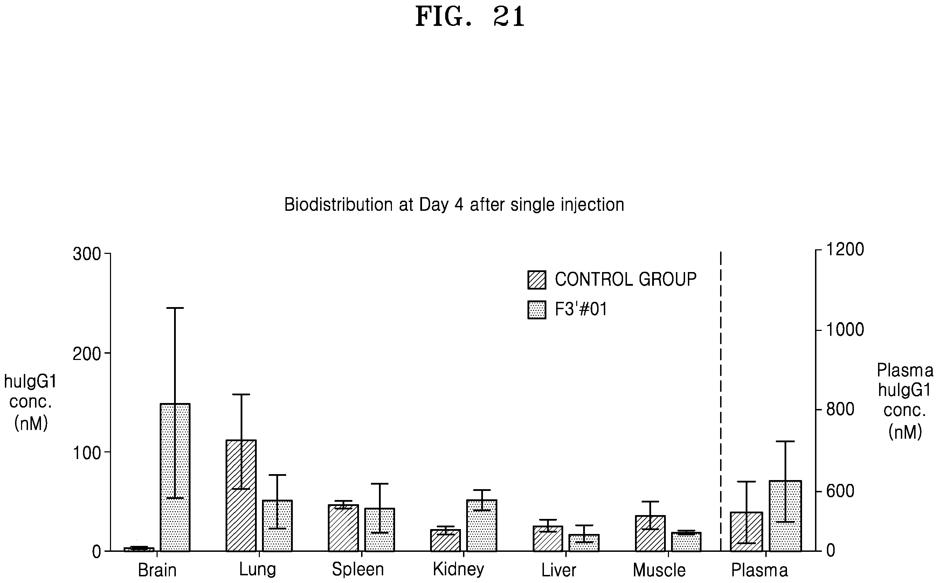

As a result, at 1 day after the intravenous administration of the BBB permeable fusion protein, F3'#01, the organ-specific distribution level of the IgG1 antibody is as shown in FIG. 20, and at 4 days after the intravenous administration of the BBB permeable fusion protein, F3'#01, the organ-specific distribution level of the IgG1 antibody is as shown in FIG. 21. In other words, it was confirmed that the F3'#01 administration group had a very high level of the IgG1 antibody distributed in the brain tissue compared to other organs.

From the results above, it was confirmed that, in the fusion protein having the functional structure according to an embodiment, unique functionality such as enhanced uptake level in brain tissue and selective delivery to brain tissue could be exhibited, even when the binding moiety to the helical region of the TfR is modified to repeat.

### 1-8. Confirmation of specific expression pattern of TfR in brain tissue

Regarding the fusion protein having the functional structure in which the tetravalent binding moiety according to an embodiment is linked to the C-termini of the light chain and the C-termini of the heavy chain of the antibody, it was experimentally confirmed as determined in Examples above that, due to high permeability to the BBB, not only was the delivery of the IgG1 antibody to brain tissue significantly enhanced, but also high biosafety was exhibited due to selective delivery to brain tissue compared to other organs. Assuming that this efficacy is due to the interaction between the binding moiety according to one embodiment and the transferrin receptor present in brain tissue, this experimental example aimed to confirm the expression pattern and characteristics of the transferrin receptor distributed in brain tissue.

### (1) Confirmation of organ-specific distribution of TfR

Blood was collected from the orbital vein or abdominal vein of a C57BL/6 mouse, and perfused with physiological saline to remove the blood. Brain tissue was extracted from the perfused mouse, and brain vessels, parenchyma, and choroid plexus were separately isolated from the brain tissue. Therefrom, total proteins were extracted by using a RIPA buffer. For other organs such as liver, lung, kidney, and spleen, a small amount of tissue was collected and total proteins was extracted therefrom by using a RIPA buffer. The extracted proteins were mixed with an SDS-PAGE sample buffer, denatured, loaded on an SDS-PAGE gel, and then transferred to a PVDF membrane. Afterwards, each protein band was identified on the membrane by using anti-TfR and a beta-actin antibody as primary antibodies, and the intensity of the protein bands was measured by using an Image J software.

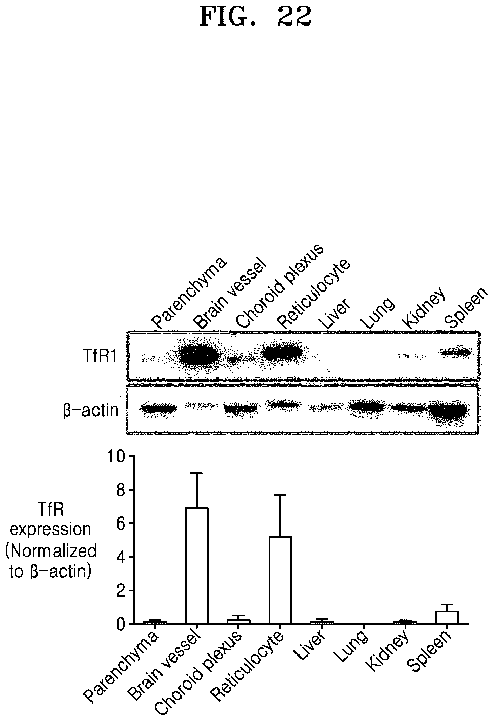

As a result, as shown in FIG. 22, it was confirmed that, as a result of quantifying the TfR expressed in each organ, the TfR was expressed in large amounts in the brain vessels and blood cells, as well as in the spleen in a significant level. The experimental results above indicate that the selective delivery of the fusion protein according to an embodiment into the brain tissue did not simply result from quantitative differences in the TfR expression in each organ, but rather may be due to interactions between the TfR with specific expression in the brain vessel and the binding moiety according to an embodiment or the function structure that can form such interactions.

### (2) Comparison of expression patterns of TfR clusters

Blood was collected from the orbital vein or abdominal vein of a C57BL/6 mouse, and perfused with physiological saline to remove the blood. Meanwhile, brain tissue was sequentially perfused with saline and 4% paraformaldehyde to prepare brain tissue samples for brain slice staining using immunohistology. Afterwards, to produce brain slices, the brain tissue was added to a mold, and a tissue freezing medium was added to sufficiently immerse the brain tissue, and then the mold was stored in a deep freezer at - 70°C for one day. Frozen brain blocks were sectioned into 40 µm-thick slices by using a cryostat. These slices were stored in a PBS solution containing 0.1% sodium azide until use.

Afterwards, the tissue slices were added to a 24-well plate and allowed for a reaction with 500 µl of a PBS solution containing 0.5% Triton-X 100 at room temperature for 20 minutes. Afterwards, the reaction solution was replaced with a PBS solution and mixed by using a plate stirrer. Afterwards, the tissue slices were added to a PBS solution containing 5% BSA and 0.1% Triton-X 100, allowed for a reaction at room temperature for 2 hours, and then washed three times with a PBS solution. Here, a TfR antibody and a CD31 antibody were added thereto to induce a reaction overnight at 4°C, and then washed three times with a PBS solution. Afterwards, anti-Rat Alexa488 and anti-Goat Alexa568 were added thereto, allowed for a reaction at room temperature for 2 hours, and then washed three times with a PBS solution. Next, a PBS solution containing DAPI was added thereto and allowed for a reaction at room temperature for 10 minutes. The tissue slices treated as described above were placed on a slide glass, and then coated with 100 µl of a mounting solution. Afterwards, the tissue slices treated as described above were covered with a cover glass and sealed.

The fluorescence images for the stained samples were obtained by using a confocal microscope. A LAS-X software was used for the photography, and the light source intensity was adjusted to UV intensity 4% and white laser power 70% (488 - 15%, 568 - 10%). Here, a 40X objective lens was used for a magnification, and with a pixel resolution of 284 nm, the images were obtained for a total thickness of 20 µm with a thickness of 1 µm per tissue slice. Also, ultra-high resolution fluorescence images for the stained samples were obtained by using an STED confocal microscope. A LAS-X software was used for the photography, and the light source intensity was adjusted to UV intensity 6% and white laser power 70% (488 - 15%, 568 - 10%). A 100X objective lens was used for magnification, and a software zoom and STED functions were used. In addition, the magnification was set with a pixel solution of 50 nm for photography, and images were obtained for a total thickness of 3 µm with a thickness of 100 nm per tissue slice.

Afterwards, the images thus obtained were analyzed by using an imaged and a MATLAB software. As a preprocessing process for analyzing the confocal fluorescence images and the STED confocal fluorescence images, a gaussian blur was performed by setting a sigma value to 1.0, each image was cropped to the same size, and a representative image was created for each color channel. To increase a signal-to-noise ratio, an imaged software plugin was utilized to perform background subtraction using a rolling ball algorithm. A green signal, which is a signal of the TfR, was normalized to an intensity histogram, and a binary image was obtained by using a local thresholding method. For the multiple masked TfR clusters, an analyze particle function was utilized to select only pixels with a minimum pixel unit of 2500 nm² or more to obtain a cluster-sized distribution plot. Such a distribution plot is representative of the distribution plot of TfRs observed in 10 cells and tissues from each organ in a total of 4 C57BL/6 mice, for a total of 40 cells per organ. Also, MATLAB was used to convert the distribution plot results obtained from the imaged into a histogram, and a smooth function was used to obtain a trend line of the histogram.

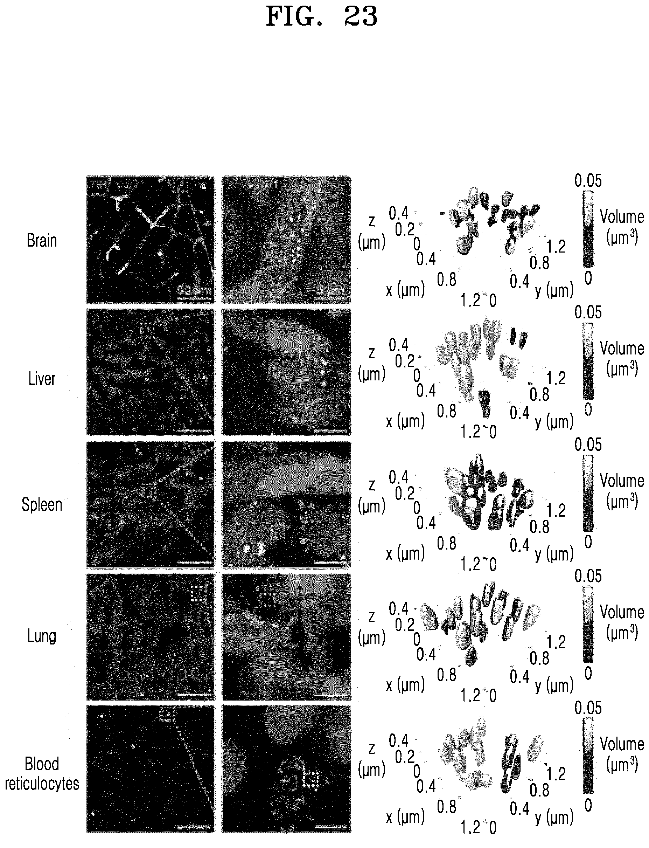

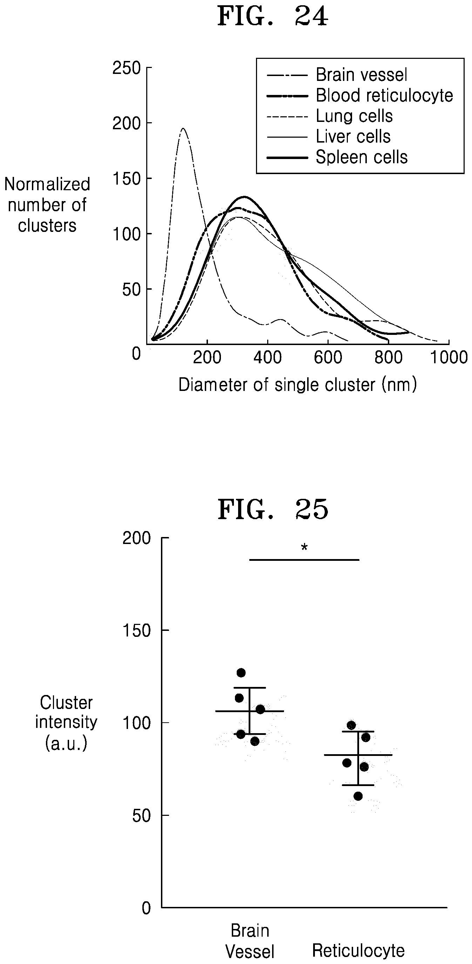

As a result, as shown in FIG. 23, it was confirmed that the TfRs expressed in brain tissue, liver, spleen, lung, and reticulocytes were distributed in the form of TfR clusters. Also, as shown in FIG. 24, it was confirmed that, as a result of comparing the quantitative distribution of the size of TfR clusters expressed in brain vessels, reticulocytes, lung, liver, and spleen, relatively small sized TfR clusters were densely present in the vascular region of brain tissue, unlike in other tissues.

### (3) Evaluation of characteristics of TfR clusters for each organ

### A. Evaluation of relative density through fluorescence intensity evaluation

To evaluate the characteristics of the TfR clusters for each organ, the average intensity value of all TfR clusters across the brain vessels and reticulocyte was evaluated. In detail, after imaging the TfR clusters in the same manner as in Section (2) of Experimental Example 1-8, the masked TfR clusters were extracted into pixels with a minimal pixel unit of 2500 nm² or more by using the analyze particle function. Afterwards, the extracted image was converted into a mask image, and the intensity of green signals in the cluster mask was calculated by using MATLAB by multiplying the mask image obtained from the imaged with the image from which the background subtraction was performed.

As a result, as shown in FIG. 25, a higher green signal intensity was observed in brain tissue vessels, indicating that the TfR clusters in brain tissue vessels were densely present with a greater number of TfRs compared to those in reticulocytes.

### B. Quantitative density evaluation through immunoblotting and image analysis

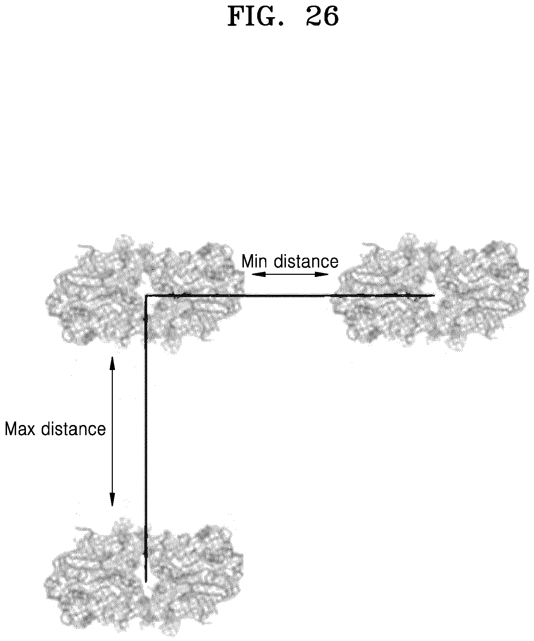

50,000 cells were collected from brain vessel tissue and reticulocytes of the C57BL/6 muse through FACS, and total proteins were extracted by using a RIPA buffer. The extracted proteins were mixed with a sample buffer, denatured, loaded on an SDS-PAGE gel, and then transferred to a PVDF membrane. Afterwards, each protein band was identified on the membrane by using anti-TfR antibody as a primary antibody, and the intensity of the protein bands was measured by using an Image lab software (Biorad). The intensity of the TfR protein bands measured in two types of cells was quantified as the number of TfRs per single cell by using a standard curve of the recombinant TfR band. Next, in order to calculate the TfR density per single cell, the average diameter and number of the TfR clusters measured by using the STED confocal microscope described above were used to calculate the total area of TfR clusters within a single cell, and the quantified number of TfRs was divided by the calculated total area to count the number of TfRs per cluster area within a single cell. The calculated number of TfRs was divided by the Avogadro number and converted to a molar concentration per area. Here, since the TfR exists as a dimer, the calculated molar concentration was converted to a halved value. Assuming that the TfRs are filled in a square lattice, the distance between the TfRs within the cluster was calculated as the distance from the center point of the dimeric TfR to the center point of the neighboring TfR, and as shown in FIG. 26, the distance from the center to the surface of the TfR is determined for the long and short axes. That is, when multiple TfRs were arranged/aligned in a lattice form, the distance between the receptor and the neighboring receptor surface centered on the long axis of the dimer was calculated as a minimum (min) value, and the distance between the receptor and the neighboring receptor centered on the short axis of the dimer was calculated as a maximum (max) value, and the average value of these two values was used for the evaluation.

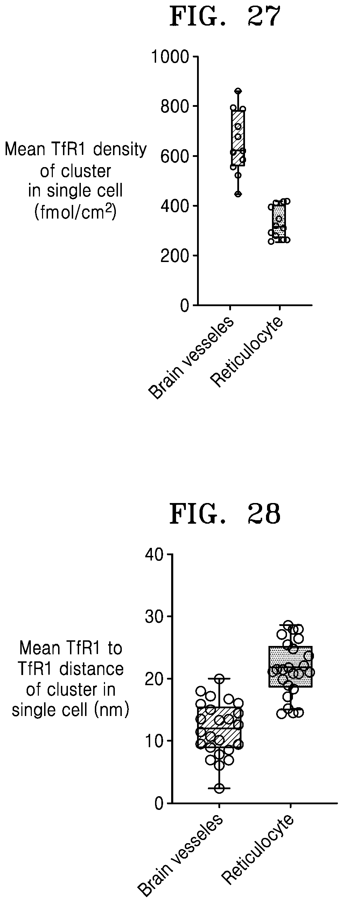

The results of evaluating the density of the total TfRs across the brain vessels and reticulocytes and the distance between the TfRs are shown in FIGS. 27 and 28. In detail, as shown in FIG. 27, the distribution of the density of the TfRs expressed in each single cell of the brain vessels was observed to be statistically significantly higher than the TfRs expressed in the reticulocytes. Also, as a result of calculating the distribution of surface-to-surface distances between the TfRs expressed within the cluster of a single cell, as shown in FIG. 28, a statistically shorter surface-to-surface distance between the TfRs of the brain vessel cells than the distance of the TfRs in the reticulocytes was observed as shown in FIG. 28. In other words, it was confirmed that the expression pattern of the TfRs inside the TfR clusters of the blood vessel cells was more dense than the TfRs in the reticulocytes.

Summarizing the experimental results above, the distribution pattern of specific TfR clusters in the vascular regions of the brain tissue shows that the tetravalent binding moiety according to an embodiment can influence interactions with the fusion protein having the fusion structure in which the binding moiety is linked to the light chain C-termini and the heavy chain C-termini of the antibody. Also, it was confirmed that the specific expression pattern of the TfR clusters that induce specific interactions in the brain tissue may act as a factor enabling permeability to the BBB and selective uptake of the IgG1 antibody into the brain tissue.

### Experimental Example 2. Evaluation of functionality of BBB permeable fusion proteins F3#02 to F3#41

In this experimental example, it was aimed to determine whether the fusion protein in which the binding moiety to the helical region of the TfR is tetravalently linked to the C-terminus regions of the light chain and the C-terminus regions of the heavy chain in the IgG1 antibody can be functional, even when the binding moiety to the helical region of the TfR is modified.

### 2-1. Evaluation of functionality of binding moiety

The functionality of the binding moiety of the BBB permeable fusion protein according to an embodiment was aimed to be determined by the binding ability to the helical region of the TfR and the evaluation of level of delivery into human brain endothelial cells accordingly.

### (1) Confirmation of binding between helical region of TfR and binding moiety

The binding between the TfR and the helical region binding moieties #02, #03, #05 to #25, #27, #30, #32, #33, #34, #36, #38, and #39 of the fusion protein prepared in Examples above was confirmed by docking simulation. The structures of these binding moieties were modeled by using a RosettaRelax program, and the position expected to interact with the helical region of the TfR was modeled by using structural information and thermodynamic calculation. Afterwards, the docking simulation that can find the most stable position by randomly changing the position of the binding moiety and calculating the interaction with the helical region was carried out by using a RosettaDocking program. 20,000 simulations were carried out for each sequence ID number, and the resulting data were analyzed based on homology to the initial modeling structure and the thermodynamic structural stability.

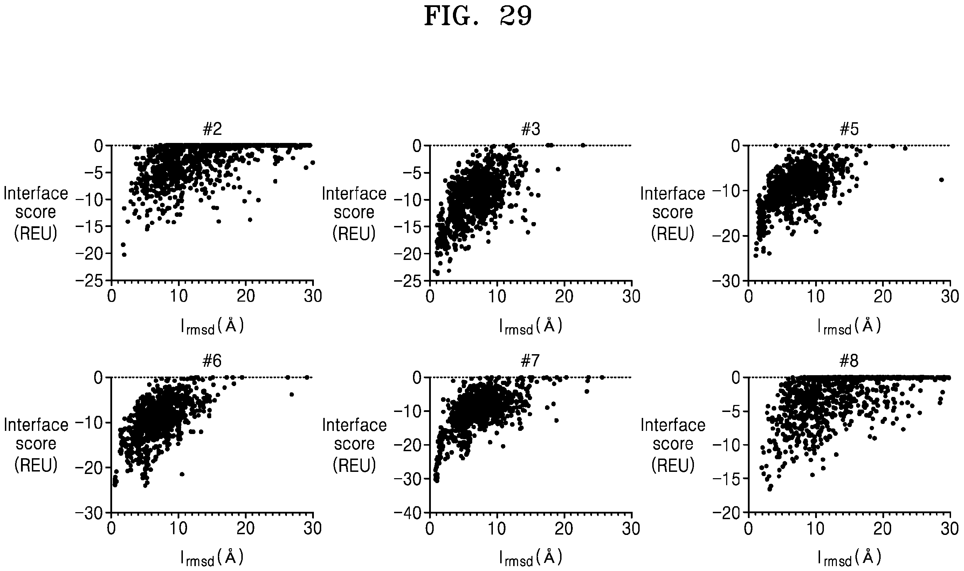

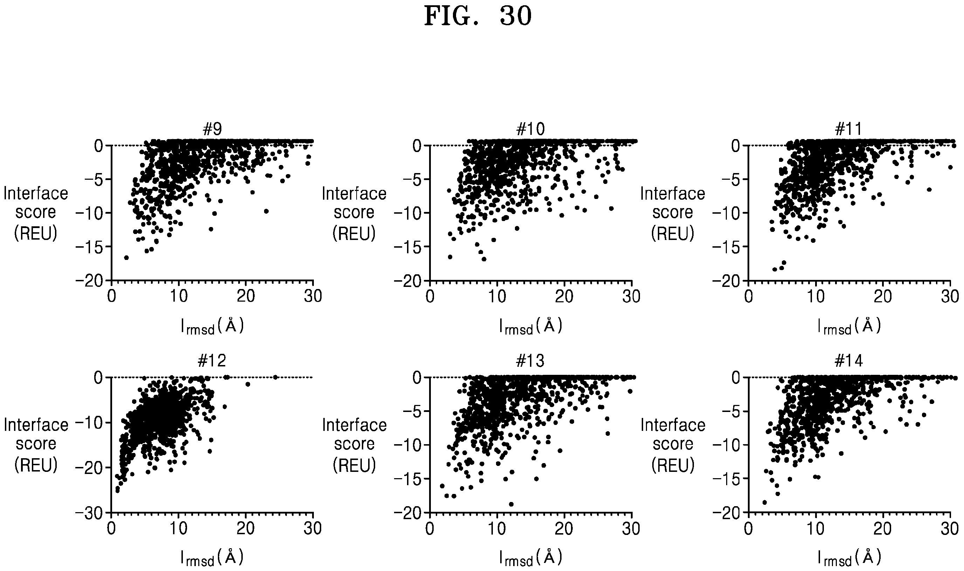

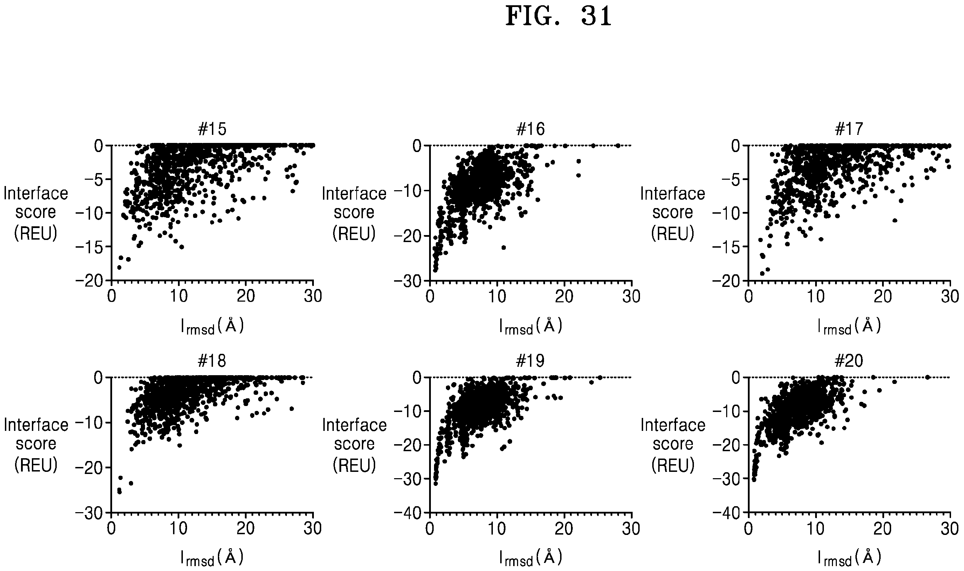

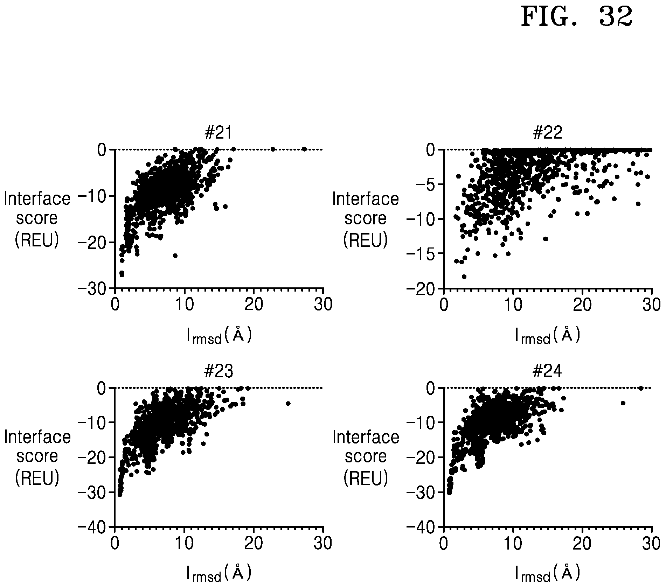

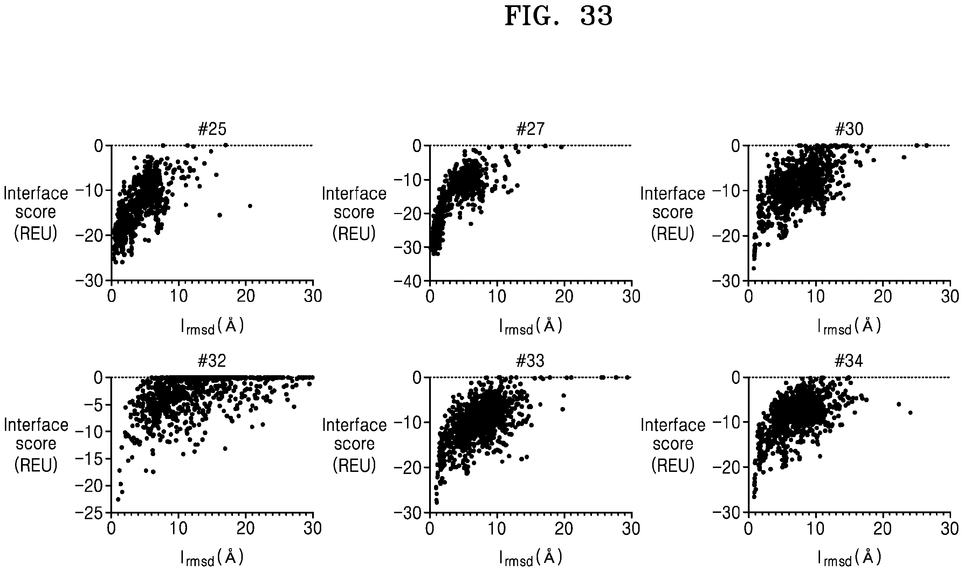

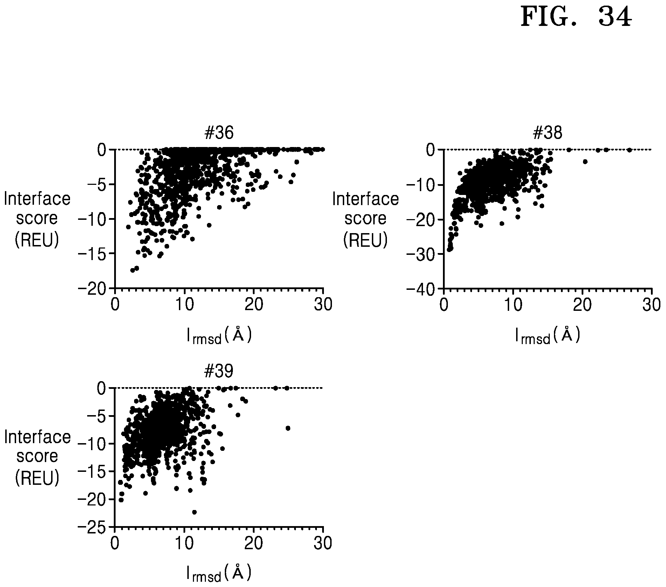

As a result, as shown in FIGS. 29 to 34, it was confirmed that the binding moieties according to an embodiment stably bound to the helical region of the TfR.

### (2) Confirmation of delivery into human brain endothelial cells

In the same manner as in Section (2) of Experimental Example 1-1, it was aimed to determine whether the fusion proteins prepared according to Examples can be delivered into hCMEC/D3 cells, which are human brain endothelial cells constituting the human BBB, through interactions between the TfRs and the helical region binding moieties #04, #16, #19, #20, #25 to #29, #31, #34 to #38, #40, and #41. Meanwhile, a group intravenously administered with the IgG1 antibody only was used as a control group.

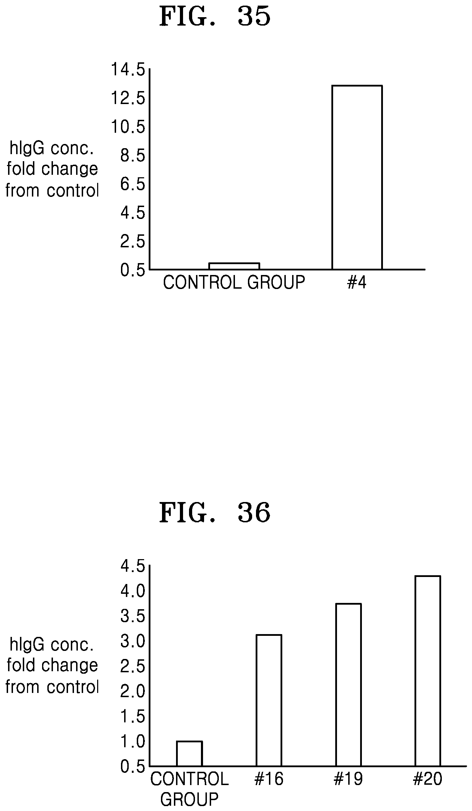

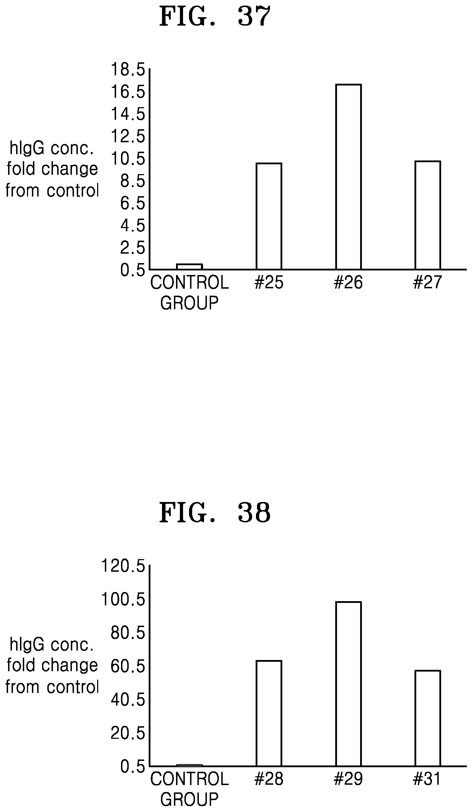

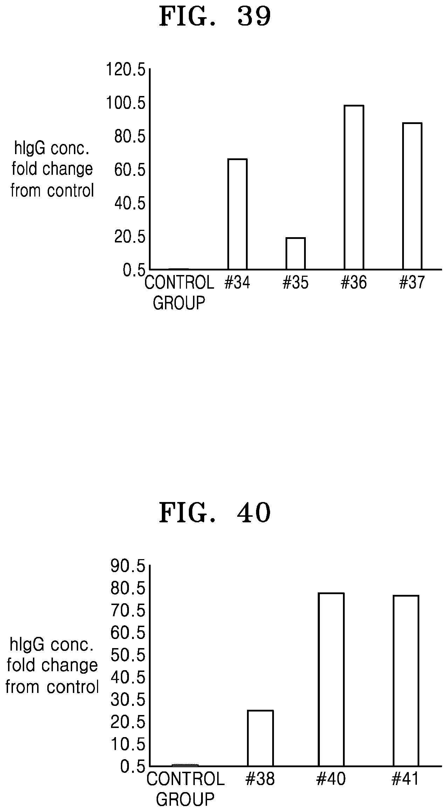

As a result, as shown in FIGS. 35 to 40, the effective delivery ability of the binding moieties according to an embodiment into the cells was confirmed as a result of evaluating the level of delivery into the cells by using the human brain endothelial cell line (hCMEC/D3).

### 2-2. Evaluation of level of permeability of fusion protein to BBB

In the same manner as in Section (1) of Experimental Example 1-2, the level of uptake of the IgG1 antibody into brain tissue by intravenous administration of the BBB permeable fusion proteins F3#02 to F3#42 according to an embodiment was evaluated at 2 or 4 days after the intravenous administration. Meanwhile, a group intravenously administered with the IgG1 antibody only was used as a control group.

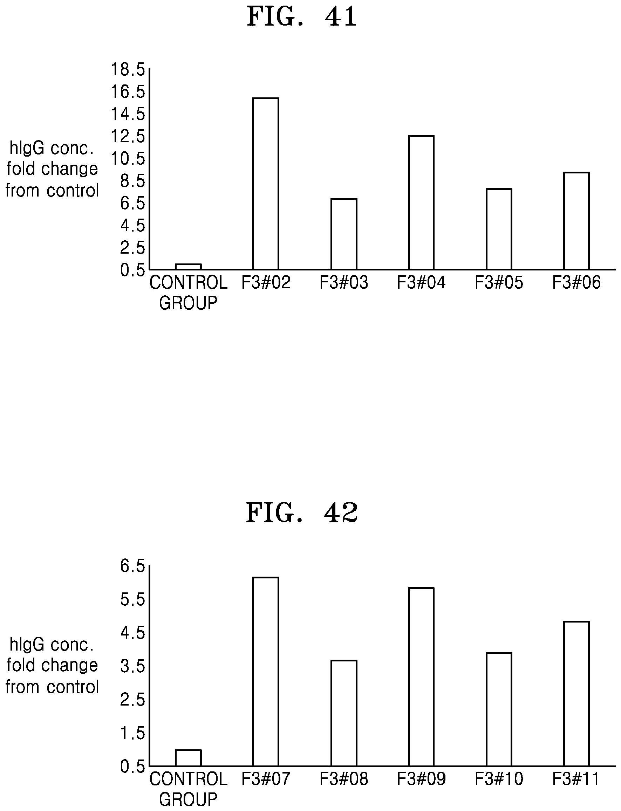

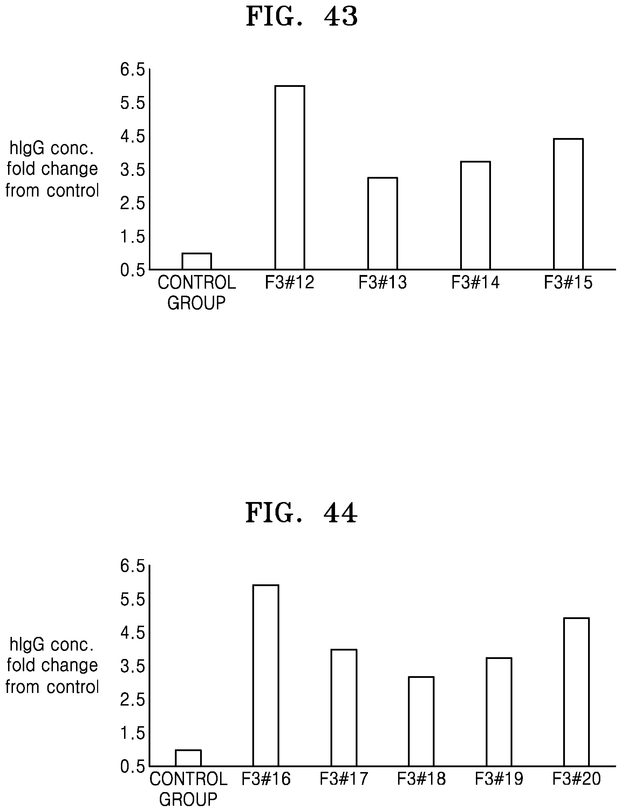

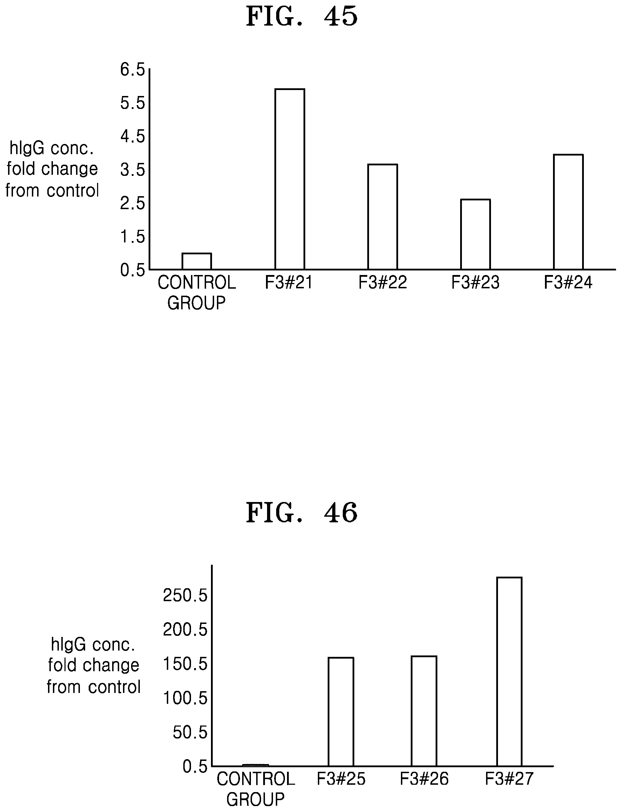

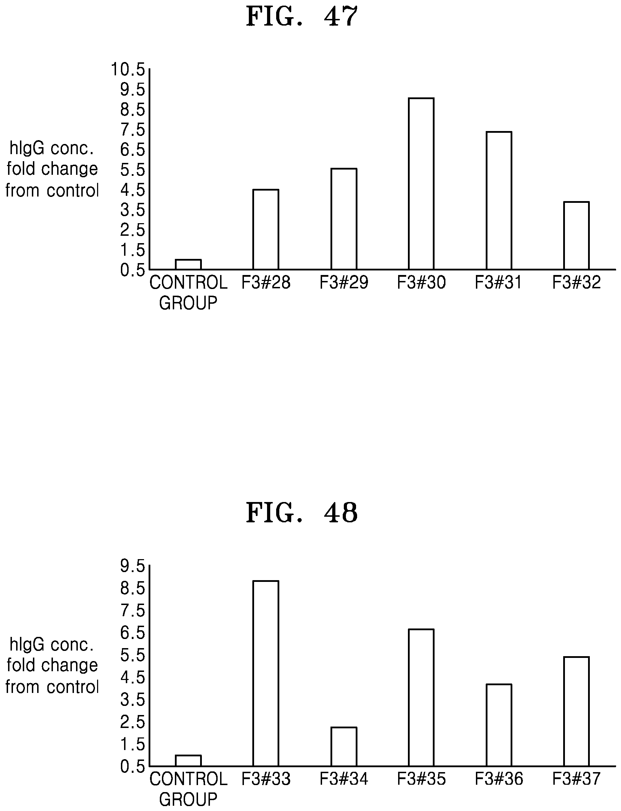

As a result, as shown in FIGS. 41 to 45, it was confirmed that all groups administered with the BBB permeable fusion proteins F3#02 to F3#24 of Examples 2 to 24, which retain the binding properties for the helical region, but are prepared by using the first binding moiety group having different amino acid sequences, showed a significant increase in the level of IgG1 antibody in the brain tissue compared to the control group. Also, as shown in FIGS. 46 to 49, it was confirmed that all groups administered with the BBB permeable fusion proteins F3#025 to F3#41 of Examples 25 to 41, which maintain at least a certain level of sequence identity to the binding moiety of SEQ ID NO: 3, but are prepared by using the second binding moiety group having substitution, insertion, or deletion of some of the amino acid sequences, showed a significant increase in the level of IgG1 antibody in the brain tissue compared to the control group.

Summarizing the above experimental results, it was confirmed that the fusion proteins according to an embodiment exhibits effects such as high permeability to the BBB and high delivery of the IgG1 antibody to brain tissue, as a function derived from a moiety with effective binding affinity for the helical region of the TfR.

### Experimental Example 3. Evaluation of functionality depending on valency change of binding moiety in BBB permeable fusion protein

In this experimental example, it was aimed to determine effects on the functionality of the aforementioned fusion proteins, when the valency of the binding moiety linked to the IgG1 was changed in the fusion protein liked with the binding moiety to the helical region of the TfR.

In detail, in the same manner as in Section (1) of Experimental Example 1-2, the level of uptake of the IgG1 antibody into brain tissue by intravenous administration of the fusion proteins was evaluated at 2 or 4 days after the intravenous administration, with respect to: 1) a fusion protein (F3) in which the binding moiety to the helical region of the TfR is tetravalently linked to the C-terminus regions of the light chain and the C-terminus regions of the heavy chain in the IgG1 antibody; 2) a fusion protein (F1) in which the binding moiety to the helical region of the TfR is divalently linked to the C-terminus regions of the heavy chain in the IgG1 antibody; and 3) a fusion protein (F5) in which the binding moiety to the helical region of the TfR is divalently linked to the C-terminus regions of the light chain in the IgG1 antibody.

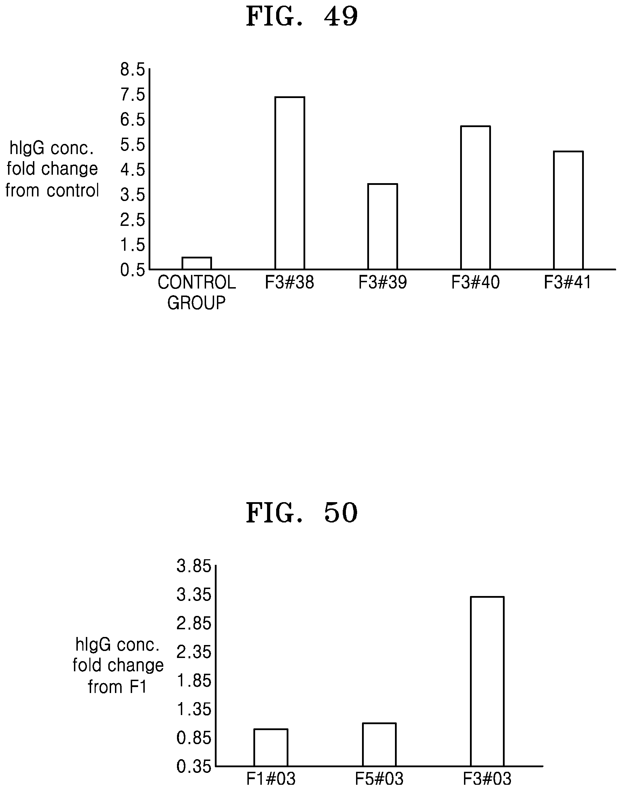

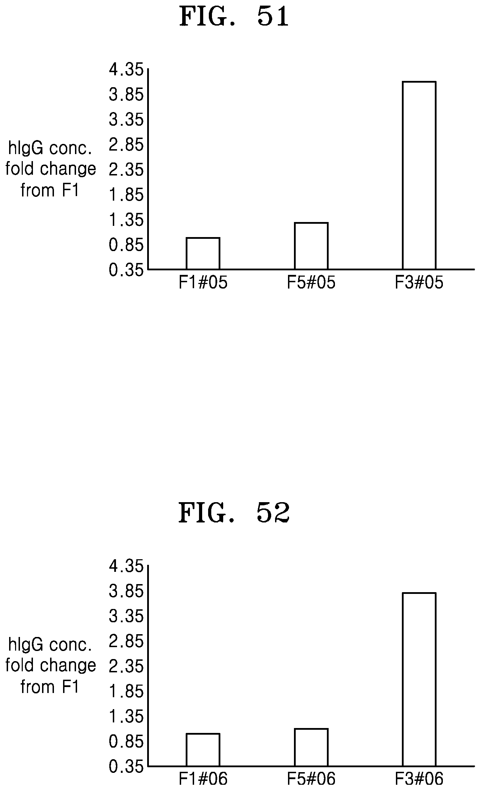

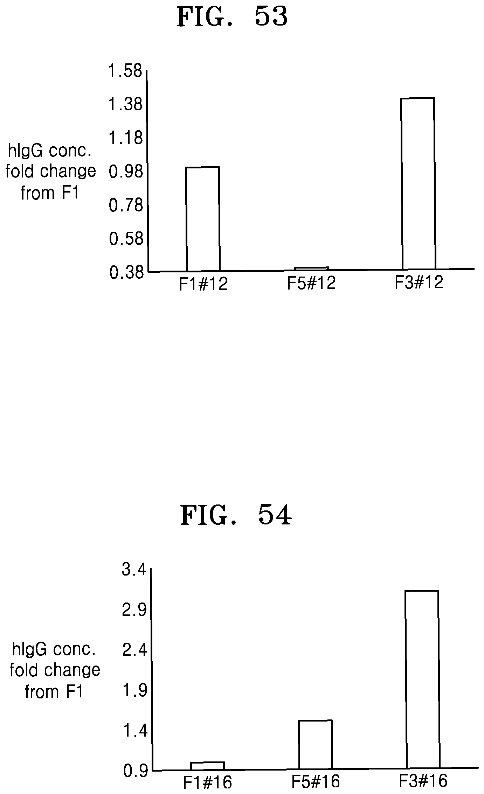

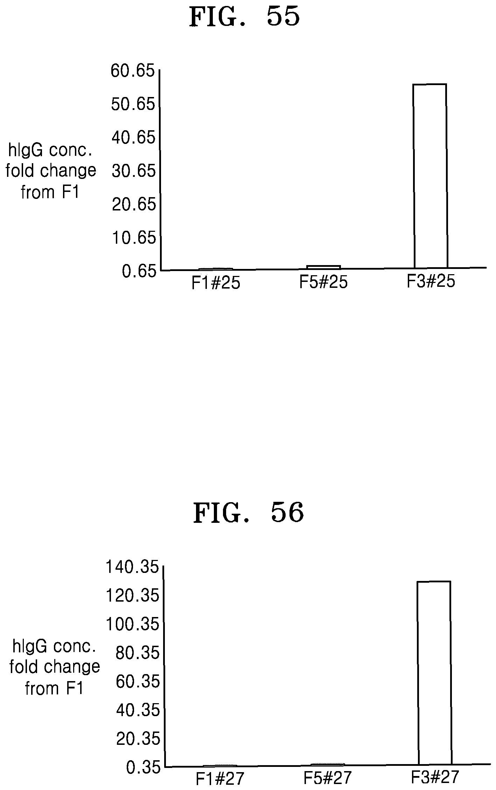

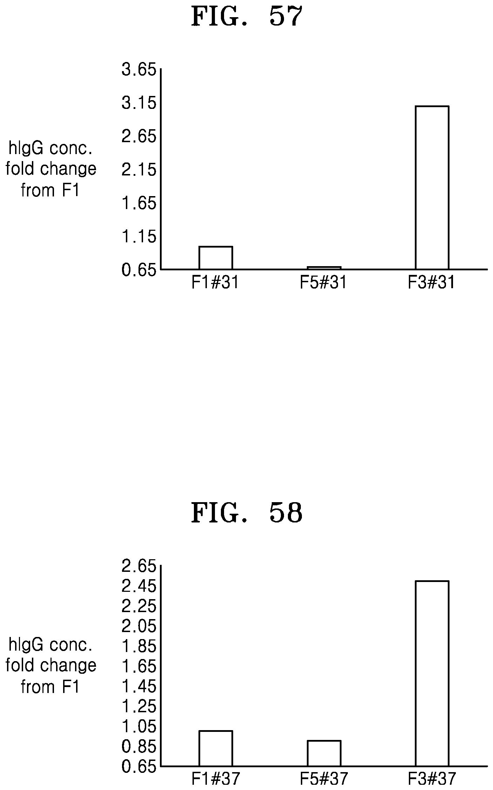

As a result, as shown in FIGS. 50 to 59, it was confirmed that, as a result of quantifying and comparing the level of the IgG1 antibody in the brain tissue, the group administered with the fusion protein in which the binding moiety is linked to each of the four regions including the heavy chain C-termini and the light chain C-termini (F3#03, F3#05, F3#06, F3#12, F3#16, F3#25, F3#27, F3#31, F3#37, or F3#40) showed a significant increase in the level of the IgG1 antibody in the brain tissue compared to the group administered with the fusion protein in which the binding moiety is linked to two regions including the heavy chain C-termini (F1#03, F1#05, F1#06, F1#12, F1#16, F1#25, F1#27, F1#31, F1#37, or F1# 40) or the group administered with the fusion protein in which the binding moiety is linked to two regions including the light chain C-termini (F5#03, F5#05, F5#06, F5#12, F5#16, F5#25, F5#27, F5 #31, F5#37, or F5#40).

Summarizing the above experimental results, it was confirmed again that since the fusion protein according to an embodiment had a structure that reflects the distribution pattern of the specific TfR clusters in the vascular region of the brain tissue as described above, the unique functionality could be exhibited when the moiety with effective binding affinity for the helical region of the TfR is tetravalently linked to the end of the IgG1 antibody.

### Experimental Example 4. Evaluation of reactivity of IgG1 antibody by linkage of binding moiety

In this experimental example, it was aimed to determine, in the fusion protein in which the binding moiety to the helical region of the TfR is tetravalently linked, effects of the linkage with the binding moiety on the reactivity, i.e., binding ability to a target, of the IgG1 antibody.

In detail, 100 ul of 0.25 ug/ml human PD-L1 protein was added to a 96 well plate, and the protein was applied onto the plate at 4°C for 16 hours. Afterwards, the coated plate was washed four times with PBS-T, 200 ul of a blocking buffer was added thereto, followed by incubation at room temperature for 2 hours, to prevent non-specific binding of the antibody. Afterwards, the plate was washed four times with PBS-T, and then 4 nM fusion proteins (F3#01 and F3'#01) were added to the well and incubated again at room temperature for 2 hours. Afterwards, 100 ul of a detection antibody, anti-human IgG FCγ HRP-conjugated antibody, was added to the well containing each sample, and then incubated at room temperature for 1 hour. Afterwards, the incubated plate was washed four times with PBS-T, and then 100 ul of TMB was added to each well and allowed for a reaction at room temperature for 10 minutes. Afterwards, 100 ul of a stop solution was added to stop the reaction, and the absorbance of each well was measured at a wavelength of 450 nm by using a microplate reader. Meanwhile, in this experimental example, an anti-PD-L1 antibody was used as the IgG1 antibody, the helical region binding moiety #01 of the TfR was used, and a group in which only the IgG1 antibody was added was used as a control group.

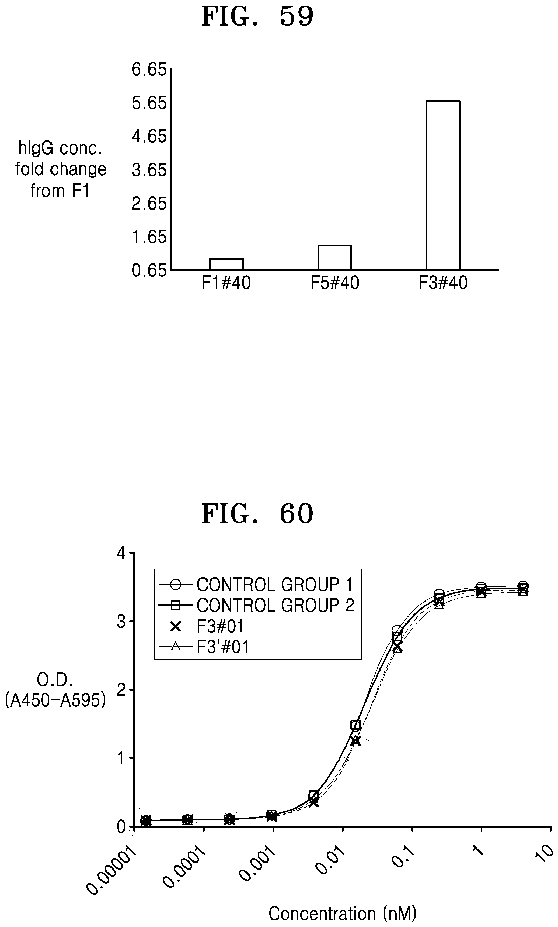

As a result, as shown in Table 2 and FIG. 60, it was confirmed that the fusion proteins F3#01 and F3'#01 according to an embodiment showed similar levels of the IgG1 antibody reactivity, i.e., binding ability to the PD-L1, as the control group. The results above suggest that the effects of permeating the BBB and selectively delivering to brain tissue by the fusion protein according to an embodiment are exhibited while retaining the biological activity inherent in the IgG antibody.

**[Table 2]**

| | **Control 1** | **Control 2** | **F3#01** | **F3'#01** |
|---|---|---|---|---|
| **Bottom** | 0.07391 | 0.1006 | 0.1029 | 0.09810 |
| **Top** | 3.521 | 3.493 | 3.457 | 3.420 |
| **Hillslope** | 1.263 | 1.230 | 1.315 | 1.230 |
| **EC₅₀ (nM)** | 0.01961 | 0.02113 | 0.02610 | 0.02585 |
| **R²** | 0.9994 | 0.9974 | 0.9993 | 0.9990 |

### Experimental Example 5. Evaluation of applicability with various IgG1 antibodies

In this experimental example, it was aimed to determine whether the fusion protein in which the binding moiety to the helical region of the TfR is tetravalently linked to the C-terminus regions of the light chain and the C-terminus regions of the heavy chain in the IgG1 antibody can be functional, even when the IgG1 antibody is modified.

### 5-1. Anti-tau IgG1 antibody

Among the fusion protein (F3) in which the binding moiety to the helical region of the TfR is tetravalently linked to the C-terminus regions of the light chain and the C-terminus regions of the heavy chain in the IgG1 antibody, an IgG1 antibody (anti-Tau) binding to Tau was used as the IgG1 antibody, and a binding moiety #25, #27, or #36 was used as the binding moiety to the helical region of the TfR, so as to prepare a BBB permeable fusion protein (F3#25-Tau, F3# 27-Tau, or F3#36-Tau) in the same manner as in Example 1. Afterwards, the level of uptake of the IgG1 antibody into the ISF by intravenous administration of the prepared fusion protein was evaluated in the same manner as in Section (2) of Experimental Example 1-2. Meanwhile, a group in which only the IgG1 antibody was added was used as a control group.

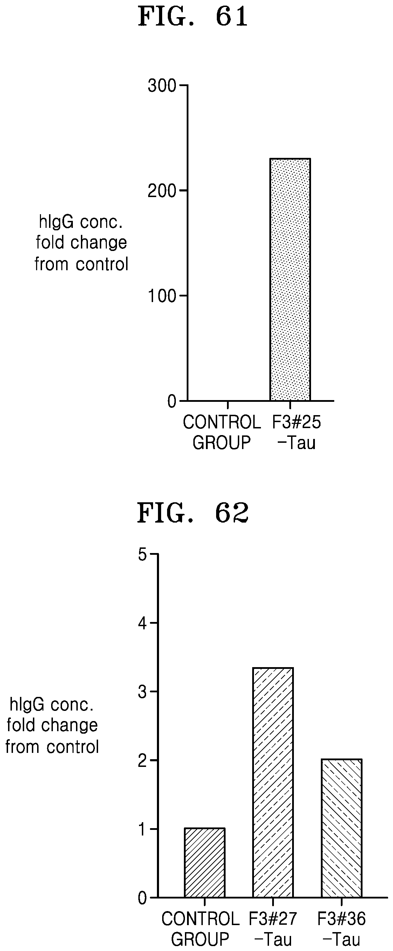

As a result, as shown in FIGS. 61 and 62, it was confirmed that the level of the anti-Tau IgG1 antibody in the ISF was increased compared to the control group, even when the type of IgG1 antibody, which is a fusion partner of the binding moiety to the helical region of the TfR, was changed to the anti-Tau IgG1 antibody while maintaining the aforementioned functional structure.

### 5-2. Anti-PD1 IgG1 antibody

Among the fusion protein (F3) in which the binding moiety to the helical region of the TfR is tetravalently linked to the C-terminus regions of the light chain and the C-terminus regions of the heavy chain in the IgG1 antibody, an IgG1 antibody (anti-PD1) binding to PD1 was used as the IgG1 antibody, and a binding moiety #25 was used as the binding moiety to the helical region of the TfR, so as to prepare a BBB permeable fusion protein (F3#25-PD1) in the same manner as in Example 1. Afterwards, the level of uptake of the IgG1 antibody into brain tissue by intravenous administration of the prepared fusion protein was evaluated in the same manner as in Section (1) of Experimental Example 1-2. Meanwhile, a group in which only the IgG1 antibody was added was used as a control group.

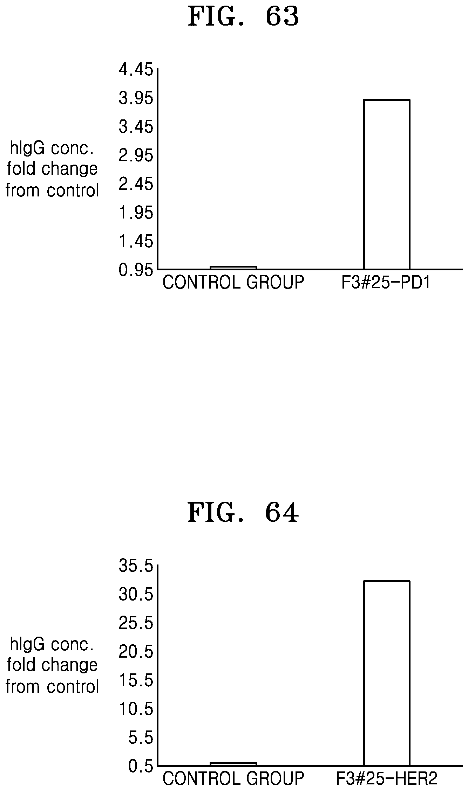

As a result, as shown in FIG. 63, it was confirmed that the level of the anti-PD1 IgG1 antibody in the brain tissue was increased compared to the control group, even when the type of IgG1 antibody, which is a fusion partner of the binding moiety to the helical region of the TfR, was changed to the anti-PD1 IgG1 antibody while maintaining the aforementioned functional structure.

### 5-3. Anti-HER2 IgG1 antibody

Among the fusion protein (F3) in which the binding moiety to the helical region of the TfR is tetravalently linked to the C-terminus regions of the light chain and the C-terminus regions of the heavy chain in the IgG1 antibody, an IgG1 antibody (anti-HER2) binding to HER2 was used as the IgG1 antibody, and a binding moiety #25 was used as the binding moiety to the helical region of the TfR, so as to prepare a BBB permeable fusion protein (F3#25-HER2) in the same manner as in Example 1. Afterwards, the level of uptake of the IgG1 antibody into brain tissue by intravenous administration of the prepared fusion protein was evaluated in the same manner as in Section (1) of Experimental Example 1-2. Meanwhile, a group in which only the IgG1 antibody was added was used as a control group.

As a result, as shown in FIG. 64, it was confirmed that the level of the anti-HER2 IgG1 antibody in the brain tissue was increased compared to the control group, even when the type of IgG1 antibody, which is a fusion partner of the binding moiety to the helical region of the TfR, was changed to the anti-HER2 IgG1 antibody while maintaining the aforementioned functional structure.

### 5-4. Anti-Aβ IgG1 antibody

Among the fusion protein (F3) in which the binding moiety to the helical region of the TfR is tetravalently linked to the C-terminus regions of the light chain and the C-terminus regions of the heavy chain in the IgG1 antibody, an IgG1 antibody (anti-Aβ) binding to Aβ was used as the IgG1 antibody, and a binding moiety #25 was used as the binding moiety to the helical region of the TfR, so as to prepare a BBB permeable fusion protein (F3#25-Aβ) in the same manner as in Example 1. Afterwards, the level of uptake of the IgG1 antibody into brain tissue by intravenous administration of the prepared fusion protein was evaluated in the same manner as in Section (1) of Experimental Example 1-2. Meanwhile, a group in which only the IgG1 antibody was added was used as a control group.

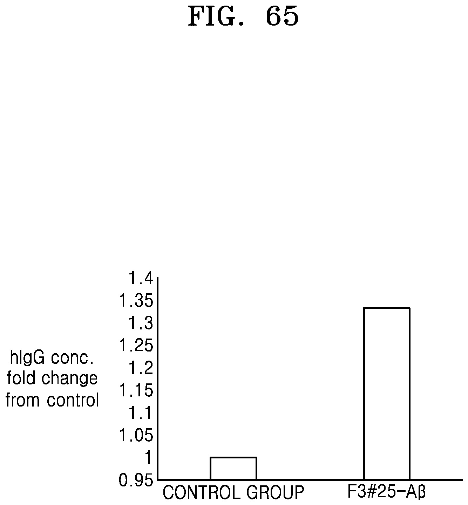

As a result, as shown in FIG. 65, it was confirmed that the level of the anti-Aβ IgG1 antibody in the brain tissue was increased compared to the control group, even when the type of IgG1 antibody, which is a fusion partner of the binding moiety to the helical region of the TfR, was changed to the anti-Aβ IgG1 antibody while maintaining the aforementioned functional structure.

From the results above, regarding a function derived from the moiety with valid binding affinity with the helical region of the TfR and the functional structure including the tetravalent moiety, it was confirmed that the fusion protein according to an embodiment exhibited effects such as high permeability to the BBB and high level of delivery of the IgG1 antibody to the brain tissue, regardless of type of the IgG1 antibody.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A blood-brain barrier permeable fusion protein comprising:
an IgG antibody; and
a tetravalent binding moiety to a helical region of a transferrin receptor (TfR) linked to a C-terminus region of a light chain and a C-terminus region of a heavy chain of the IgG antibody.

2. The fusion protein of claim 1, wherein the blood-brain barrier permeable fusion protein forms a complex by binding to the TfR that forms TfR clusters distributed specifically in blood vessels of the blood-brain barrier.

3. The fusion protein of claim 1, wherein the blood-brain barrier permeable fusion protein is delivered selectively to a brain tissue.

4. The fusion protein of claim 1, wherein the binding moiety has binding affinity for at least one amino acid selected from a helical region of SEQ ID NO: 2 in the TfR.

5. The fusion protein of claim 1, wherein a plurality of the binding moieties are the same as or different from each other.

6. The fusion protein of claim 1, wherein the IgG antibody is IgG1, IgG2, IgG3, or IgG4.

7. The fusion protein of claim 1, wherein the binding moiety is linked, via linker peptides, to the C-terminus region of the light chain and the C-terminus region of the heavy chain of the IgG antibody.

8. A polynucleotide encoding the fusion protein of any one of claims 1 to 7.

9. A vector comprising the polynucleotide of claim 8.

10. A transfection cell line transfected with the vector of claim 9.
